# EUROPEAN PATENT SPECIFICATION

(11) **EP 4 328 054 B1**
(45) Date of publication and mention of the grant of the patent: **28.01.2026**
(21) Application number: 21940171.8
(22) Date of filing: 20.05.2021
(51) Int. Cl.: B60H 3/00, A61L 9/00, B05B 12/14, B05B 17/00, B05B 17/06

(54) **CONTROL METHOD AND APPARATUS FOR AROMA ADJUSTMENT SYSTEM**
STEUERUNGSVERFAHREN UND VORRICHTUNG FÜR EIN AROMAEINSTELLUNGSSYSTEM
PROCÉDÉ ET APPAREIL DE COMMANDE POUR UN SYSTÈME D'AJUSTEMENT D'ARÔME

(43) Date of publication of application: 28.02.2024
(60) Divisional application: 25224292.0
(73) Proprietor: Shenzhen Yinwang Intelligent Technologies Co., Ltd., Shenzhen, Guangdong 518129 (CN)
(72) Inventor: WANG, Peizhi, Shenzhen, Guangdong 518129 (CN); WANG, Yi, Shenzhen, Guangdong 518129 (CN); ZHENG, Minghui, Shenzhen, Guangdong 518129 (CN)
(74) Representative: Thun, Clemens
(86) International application number: PCT/CN2021/094906
(87) International publication number: WO 2022/241716

(56) References cited:
- WO-A1-2018/147838
- WO-A1-2018/147838
- CN-A- 102 830 723
- CN-A- 102 830 723
- CN-A- 107 773 777
- CN-A- 107 773 777
- CN-A- 108 514 651
- CN-A- 108 514 651
- CN-A- 108 638 804
- CN-A- 108 638 804
- CN-A- 109 334 405
- CN-A- 109 334 405
- CN-A- 109 334 406
- CN-A- 109 367 362
- CN-A- 109 910 564
- CN-A- 109 968 949
- CN-A- 110 171 271
- CN-A- 110 412 907
- CN-A- 110 412 907
- CN-A- 110 510 250
- CN-B- 109 367 362
- CN-B- 110 171 271
- US-A1- 2020 242 421

## Description

### TECHNICAL FIELD

This application relates to the field of intelligent vehicle technologies, and in particular, to a control method and a control apparatus for an odor adjustment system.

### BACKGROUND

At present, for example, many vehicles are equipped with fragrance systems. The fragrance system emits aromas into the vehicle compartment to keep the air inside the vehicle clean or create a more comfortable atmosphere inside the vehicle, to enhance the texture and luxury of the vehicle. However, the conventional fragrance system only improves the odor in the vehicle, and user experience is single, that is, there is still room for improvement in terms of user experience.
The document CN 108 514 651 A shows a vehicle odor generating device, a vehicle odor generating system and an according application.
The document CN 107 773 777 A shows a fragrance spraying method and device.
The document CN 108 638 804 A shows a vehicle odor generation system and its application.
The document CN 102 830 723 A shows a control device, a terminal, and an according method. Especially, the control of odor release is shown.
The document CN 109 334 405 A shows a fragrance control method, and an according system for a vehicle.
The document CN 110 412 907 A shows a fragrance generator, a vehicle fragrance control system, and an according method.
The document CN 109 367 362 B shows a control device and a method for vehicle-mounted fragrance emission.
The document CN 110 171 271 B shows an automobile fragrance control method and device.
The document WO 2018/147838 A1 shows a system and method for machine learning for olfactory mood alteration.
The document US 2020/0242421 A1 shows a multi-sensor data fusion for automotive systems.

### SUMMARY

The present invention is defined by the independent claims. Further advantageous developments are shown by the dependent claims.

This application provides a control method and a control apparatus for an odor adjustment system, a computer-readable storage medium, a computing device, and the like, to improve user experience.

According to the invention, there is provided a control method for an odor adjustment system, including: obtaining scene information; determining a target odor type of a to-be-released gas based on the scene information; and sending a control instruction, where the control instruction includes a gas release instruction for releasing a gas of the target odor type.

According to the foregoing method, the target odor type of the to-be-released gas is determined based on the scene information, and a control instruction is generated, to release the to-be-released gas of the determined target odor type, to adjust an odor of an environmental space. In this way, different odor types are released in different scenes, thereby improving user experience.

The foregoing odor adjustment system may be applied to odor adjustment in a plurality of environmental spaces, for example, in a vehicle or in a home room.
In a possible implementation of the first aspect, the scene information includes one or more of driving fatigue scene information, user emotion information, off-odor removal scene information, schedule reminder information, driving mode information, music scene information, environment information, historical scene record information, or user-defined scene information.

According to the invention, the scene information comprises schedule reminder information.

According to the invention, the scene information includes schedule reminder information, the control method specifically includes: obtaining the schedule reminder information, where the schedule reminder information includes reminder content; determining a first target odor type of a first to-be-released gas based on the reminder content; and sending a first control instruction, where the first control instruction includes a first gas release instruction for releasing the first to-be-released gas of the first target odor type.

The schedule reminder information further includes a reminder time; and the control method further specifically includes: obtaining current time information; and sending the first control instruction when the reminder time matches the current time information.

In the foregoing manner, for example, when the schedule reminder information is "Meeting at 14:00", when a current time is close to 14:00 (the reminder time matches the current time information), a fragrance gas with refreshing effect may be released. In this way, user experience can be improved.

In a possible implementation of the first aspect, the method further includes: determining a target person based on the reminder content information; and sending the first control instruction when the target person is detected.

In the foregoing manner, for example, when the reminder content is "Marriage anniversary", it may be determined that the target person is a spouse of the user. When it is detected that the spouse of the user appears, a fragrance gas of a rose odor type may be released. In this way, user experience can be improved.

In a possible implementation of the first aspect, the detecting the target person specifically includes: detecting the target person based on image information and/or audio information.

When the scene information includes the driving mode information, for example, when it is determined, based on the driving mode information, that the vehicle is in a comfortable driving mode, a gas whose odor type is a fragrance of flowers may be released. In this way, user experience can be improved.

In a possible implementation of the first aspect, the user emotion information is obtained based on one or more of voice information, operation action information, or face image information.

In a possible implementation of the first aspect, when the scene information includes the off-odor removal scene information, the control method specifically includes: when the off-odor removal scene information is detected, send a second control instruction, where the second control instruction includes a second gas release instruction for releasing a deodorizing gas; and the off-odor removal scene information includes information indicating one or more of the following: receiving a deodorization instruction of a user, receiving deodorization confirmation information of the user, detecting an off-odor, detecting a deodorization requirement, and detecting a first user action.

In a possible implementation of the first aspect, the deodorization instruction of the user includes one or more of the following: a voice deodorization instruction of the user, a gesture deodorization instruction, and a deodorization instruction generated by the user through a button or a selection operation on a touchscreen;
the off-odor includes one or more of the following detected by using a smoke sensor or an off-odor sensor: a cigarette odor, a cooking fume odor, a hot pot odor, a barbecue odor, and an air conditioner off-odor; and the detected deodorization requirement includes a deodorization requirement predicted based on user information, where the user information includes one or more of the following: consumption information and location information;
the deodorization confirmation information of the user includes voice confirmation information, gesture confirmation information, or confirmation information generated through a button or a selection operation on the touchscreen that is fed back by the user after a deodorization request is sent to the user; and
the first user action includes one or more of the following: a nose covering action, a fanning action, and a window opening action.

In a possible implementation of the first aspect, the control instruction further includes an image playing instruction, and the image playing instruction is used to play an image that uses the to-be-released gas of the target odor type as a carrier.

In the foregoing manner, when a fragrance gas is released, an image is played at the same time, so that user experience can be improved. In addition, the image is played by using the to-be-released gas of the target odor type as the carrier, and no image playing carrier needs to be additionally disposed, so that manufacturing costs can be reduced.

In a possible implementation of the first aspect, the method further includes: obtaining content information of the image based on the target odor type.

In the foregoing manner, the content information of the image is obtained based on the target odor type, so that content of the played image corresponds to the target odor type. For example, when a gas of a rose odor type is released, an image of a rose is played. In this way, user experience can be improved.

In a possible implementation of the first aspect, the first control instruction further includes a first image playing instruction, and the first image playing instruction is used to play a first image that uses the released first to-be-released gas of the first target odor type as a carrier.

In a possible implementation of the first aspect, the method further includes: obtaining content information of the first image based on the first target odor type.

In a possible implementation of the first aspect, the second control instruction further includes a second image playing instruction, and the second image playing instruction is used to play a second image that uses the released second to-be-released gas of the second target odor type as a carrier.

In a possible implementation of the first aspect, the method further includes: obtaining content information of the second image based on the second target odor type.

In a possible implementation of the first aspect, before the obtaining scene information, the method further includes: obtaining residual amount information of at least one type of spice in a spice accommodating mechanism, and when a residual amount indicated by the residual amount information is less than a first threshold, sending residual amount deficiency alarm information.

In the foregoing manner, a user can learn in a timely manner of a case in which spices are insufficient, and the user is reminded to replace the spices, thereby improving user experience.

In a possible implementation of the first aspect, the method further includes: detecting verification information of a spice, and when the verification information meets a first condition, sending alarm information, where the first condition includes one or more of the following: the verification information is inconsistent with preset verification information, and the verification information cannot be read.

In the foregoing manner, when the verification information does not match the preset verification information or the verification information cannot be read, alarm information is sent, so that incorrect use of a spice that does not match a specification can be avoided, and user experience can be improved.

In a possible implementation of the first aspect, the user-defined scene information is set through a man-machine interaction interface.

According to the invention, there is provided a control apparatus for an odor adjustment system, including an obtaining module and a processing module, where the obtaining module is configured to obtain scene information; and the processing module is configured to: determine a target odor type of a to-be-released gas based on the scene information, and send a control instruction, where the control instruction includes a gas release instruction for releasing a gas of the target odor type.

In a possible implementation of the second aspect, the scene information includes one or more of driving fatigue scene information, driving mode information, music scene information, or environment information.

According to the invention, the scene information includes schedule reminder information, the obtaining module is specifically configured to obtain the schedule reminder information, where the schedule reminder information includes reminder content; and the processing module is specifically configured to: determine a first target odor type of a first to-be-released gas based on the reminder content; and send a first control instruction, where the first control instruction includes a first gas release instruction for releasing the first to-be-released gas of the first target odor type.

The schedule reminder information further includes a reminder time; and the obtaining module is further specifically configured to obtain current time information; and the processing module is specifically configured to send the first control instruction when the reminder time matches the current time information.

In a possible implementation of the second aspect, the processing module is further configured to determine a target person based on the reminder content information; and the processing module is further configured to send the first control instruction when the target person is detected.

In a possible implementation of the second aspect, when being configured to detect the target person, the processing module is specifically configured to detect the target person based on image information and/or audio information.

In a possible implementation of the second aspect, the user emotion information is obtained based on one or more of voice information, operation action information, or face image information.

In a possible implementation of the second aspect, when the scene information includes the off-odor removal scene information, the processing module is specifically configured to: when the off-odor removal scene information is detected, send a second control instruction, where the second control instruction includes a second gas release instruction for releasing a deodorizing gas; and the off-odor removal scene information includes information indicating one or more of the following: receiving a deodorization instruction of a user, receiving deodorization confirmation information of the user, detecting an off-odor, detecting a deodorization requirement, and detecting a first user action.

In a possible implementation of the second aspect, the deodorization instruction of the user includes one or more of the following: a voice deodorization instruction of the user, a gesture deodorization instruction, and a deodorization instruction generated by the user through a button or a selection operation on a touchscreen.

The off-odor may include one or more of the following detected by using a smoke sensor or an off-odor sensor: a cigarette odor, a cooking fume odor, a hot pot odor, a barbecue odor, and an air conditioner off-odor; and the detected deodorization requirement may specifically include a deodorization requirement predicted based on user information, where the user information may include one or more of the following: consumption information and location information.

The deodorization confirmation information of the user may specifically include voice confirmation information, gesture confirmation information, or confirmation information generated through a button or a selection operation on the touchscreen that is fed back by the user after a deodorization request is sent to the user.

The first user action may specifically include one or more of the following: a nose covering action, a fanning action, and a window opening action.

In a possible implementation of the second aspect, the control instruction further includes an image playing instruction, and the image playing instruction is used to play an image that uses the to-be-released gas of the target odor type as a carrier.

In a possible implementation of the second aspect, the processing module is further configured to obtain content information of the image based on the target odor type.

In a possible implementation of the second aspect, the user-defined scene information is set through a man-machine interaction interface.

In a possible implementation of the second aspect, the obtaining module is further configured to: before obtaining the scene information, obtain residual amount information of the at least one type of spice, and the processing module is further configured to: when a residual amount indicated by the residual amount information is less than a first threshold, send residual amount deficiency alarm information.

In a possible implementation of the second aspect, the processing module is further configured to: detect verification information of a spice, and when the verification information meets a first condition, send alarm information, where the first condition includes one or more of the following: the verification information is inconsistent with preset verification information, and the verification information cannot be read.

A technical effect brought by the control apparatus provided in the second aspect of this application and any possible implementation of the second aspect of this application is the same as a technical effect brought by the control method provided in the first aspect of this application and any possible implementation of the first aspect of this application. For brevity, details are not described herein again.

According to the invention, there is provided a computer-readable storage medium, where the comnuter readable storage medium stores program instructions and when the program instructions are executed by a computer, the computer performs the control method.

These aspects and another aspect of this application are clearer and easier to understand in descriptions of the following embodiments (a plurality of embodiments).

### BRIEF DESCRIPTION OF DRAWINGS

The following further describes features of this application and a relationship between the features with reference to the accompanying drawings. The accompanying drawings are all examples, and some features are not shown in actual proportions. In addition, in some accompanying drawings, common features that are not mandatory for this application in the field of this application may be omitted. Alternatively, additional features that are not mandatory for this application are not shown. A combination of the features shown in the accompanying drawings is not intended to limit this application. In addition, in this specification, content referred to by same reference signs is also the same. The specific accompanying drawings are described as follows:
FIG. 1 is a schematic illustration diagram of an example of an application scenario of an odor adjustment system according to an embodiment of this application;
FIG. 2 is a schematic block diagram of a structure of an odor adjustment system according to an embodiment of this application;
FIG. 3 is a schematic flowchart of a control method for an odor adjustment system according to an embodiment;
FIG. 4 shows a control method for determining an odor type based on scene information according to an embodiment of this application;
FIG. 5 is a schematic illustration diagram of implementation logic of an odor adjustment system according to an embodiment;
FIG. 6 is a schematic illustration diagram of a network architecture of an intelligent digital vehicle platform according to an embodiment;
FIG. 7 is a schematic illustration diagram of an application scenario of an odor adjustment system according to an embodiment of this application;
FIG. 8 is a schematic block diagram of a structure of an electronic control unit according to an embodiment of this application;
FIG. 9 is a schematic diagram of a three-dimensional structure of an odor adjustment apparatus according to an embodiment of this application;
FIG. 10 is a schematic diagram of another three-dimensional structure of an odor adjustment apparatus according to an embodiment of this application;
FIG. 11 is a schematic diagram of a three-dimensional structure of a first housing equipped with a spice container according to an embodiment of this application;
FIG. 12 is a schematic diagram of another three-dimensional structure of a first housing according to an embodiment of this application;
FIG. 13 is a schematic diagram of a three-dimensional structure of a second housing according to an embodiment of this application;
FIG. 14 is a schematic diagram of another three-dimensional structure of a second housing according to an embodiment of this application;
FIG. 15 is a schematic diagram of still another three-dimensional structure of a second housing according to an embodiment of this application;
FIG. 16 is a schematic sectional view of a second housing according to an embodiment of this application;
FIG. 17 is a schematic sectional view of a second housing according to an embodiment of this application;
FIG. 18 is a schematic diagram of a three-dimensional structure of a spice container according to an embodiment of this application;
FIG. 19 is a schematic diagram of a three-dimensional structure of a container cover of a spice container according to an embodiment of this application;
FIG. 20 is a schematic sectional view of a container cover of a spice container according to an embodiment of this application;
FIG. 21 is a schematic diagram of a three-dimensional structure of a container body of a spice container according to an embodiment of this application;
FIG. 22 is a schematic diagram of another three-dimensional structure of a container body of a spice container according to an embodiment of this application;
FIG. 23 is a schematic illustration diagram of an installation structure of an odor adjustment apparatus according to an embodiment of this application;
FIG. 24 is a schematic diagram of a structure of a mounting plate of an odor adjustment apparatus according to an embodiment of this application;
FIG. 25 is a schematic illustration diagram of another installation structure of an odor adjustment apparatus according to an embodiment of this application;
FIG. 26 is a schematic diagram of a structure of a mounting base of an odor adjustment apparatus according to an embodiment of this application;
FIG. 27 is a three-dimensional schematic diagram of an odor adjustment apparatus according to an embodiment of this application;
FIG. 28 is a schematic sectional view of an image playing unit according to an embodiment of this application;
FIG. 29 is a three-dimensional schematic diagram of an odor adjustment apparatus according to an embodiment of this application;
FIG. 30 is another three-dimensional schematic diagram of an odor adjustment apparatus according to an embodiment of this application;
FIG. 31 is still another three-dimensional schematic diagram of an odor adjustment apparatus according to an embodiment of this application;
FIG. 32 is a three-dimensional schematic diagram of a spice container according to an embodiment of this application;
FIG. 33 is another three-dimensional schematic diagram of a spice container according to an embodiment of this application; and
FIG. 34 is a block diagram of a structure of a control apparatus according to an embodiment of this application.

### DESCRIPTION OF EMBODIMENTS

The following describes specific implementations of this application with reference to accompanying drawings.

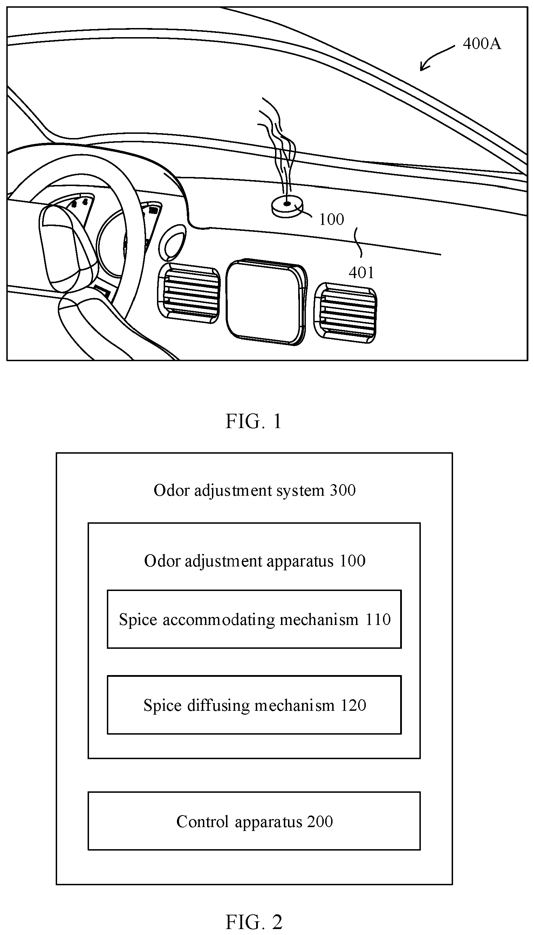

FIG. 1 is a schematic illustration diagram of an example of an application scenario according to this application. As shown in FIG. 1, an odor adjustment apparatus 100 is installed on a front window sill plate 401 of a vehicle 400A. Under control of a control apparatus (not shown in the figure), the odor adjustment apparatus 100 emits an aroma gas to a compartment. This can adjust an odor in the compartment, and can create a good atmosphere in the vehicle.

FIG. 2 is a schematic block diagram of a structure of an odor adjustment system according to an embodiment of this application. As shown in FIG. 2, the odor adjustment system 300 includes an odor adjustment apparatus 100 and a control apparatus 200.

The odor adjustment apparatus 100 may be disposed on a vehicle, as shown in FIG. 1, or may be disposed indoors, and is configured to release an aroma gas to an environmental space (a target space for odor adjustment) such as a compartment or indoors, to adjust an odor in the environmental space and create a good gas atmosphere.

The odor adjustment apparatus 100 includes a spice accommodating mechanism 110 and a spice diffusing mechanism 120. The spice accommodating mechanism 110 is configured to accommodate a spice, and may include, for example, an accommodating bottle or an accommodating box. The spices can be liquid, solid, or paste. In addition, the spice accommodating mechanism 110 may have a plurality of accommodating bottles or accommodating boxes, or the accommodating bottles or accommodating boxes have a plurality of accommodating cavities, to accommodate a plurality of types of spices, and can emit a plurality of types of aroma gases to an environmental space. These types include a gas type formed by a single type of spice and a gas type formed by mixing a plurality of types of spices.

The spice diffusing mechanism 120 is configured to diffuse the spices accommodated by the spice accommodating mechanism 110 to the environmental space. A specific structure of the spice diffusing mechanism 120 may be of a plurality of forms, which is not limited in this application. For example, the structure may be a structure in which a fan or an air pump is used to blow the spices to the environmental space, or may be a structure in which the spices are diffused to the environmental space by using an ultrasonic atomization principle, or may be a structure in which the spices are diffused to the environmental space by using a dual-fluid atomization principle, or may be a structure in which the spices are diffused to the environmental space in a heating manner.

In addition, the spice diffusing mechanism 120 is formed to diffuse the plurality of types of spices to the environmental space separately. Herein, "diffuse to the environmental space separately" means that only one type of spice is diffused to the environmental space at a same moment. In addition, the spice diffusing mechanism 120 may be further formed to diffuse the plurality of types of spices to the environmental space separately, and diffuse any combination of the plurality of types of spices to the environmental space simultaneously. A function of "diffuse to the environmental space separately" may be implemented by separately setting a structure for diffusing the plurality of types of spices, and the function of "diffuse any combination of the plurality of types of spices to the environmental space simultaneously" may be implemented by adding, based on the foregoing structure, a structure that mixes gases formed by diffusing different spices. "diffuse to the environmental space separately" may also be referred to as separately diffusing, and "diffuse to the environmental space simultaneously" may also be referred to as mixedly diffusing.

The control apparatus 200 is configured to control the odor adjustment apparatus 100, and specifically, control the spices to be diffused to the environmental space by controlling the spice diffusing mechanism 120. In an embodiment, the control apparatus 200 may be implemented by using an electronic control unit (Electronic Control Unit, ECU). In addition, the control apparatus 200 may be disposed independently of the odor adjustment apparatus 100 in a physical space, or may be integrated with the odor adjustment apparatus 100.

In addition, in addition to being automatically controlled by the control apparatus 200, diffusing of the spices to the environmental space may also be actively controlled by a user. For example, a corresponding control button is disposed on the odor adjustment apparatus 100. The control button includes an on/off button, an aroma mode adjustment button, and the like. The aroma mode adjustment button is used to adjust an odor type of a to-be-released gas. In another embodiment, the on/off button and the aroma mode adjustment button may also be software buttons existing in the control apparatus. For example, the on/off button and the aroma mode adjustment button are provided on a man-machine interaction interface displayed on a central display screen of a vehicle.

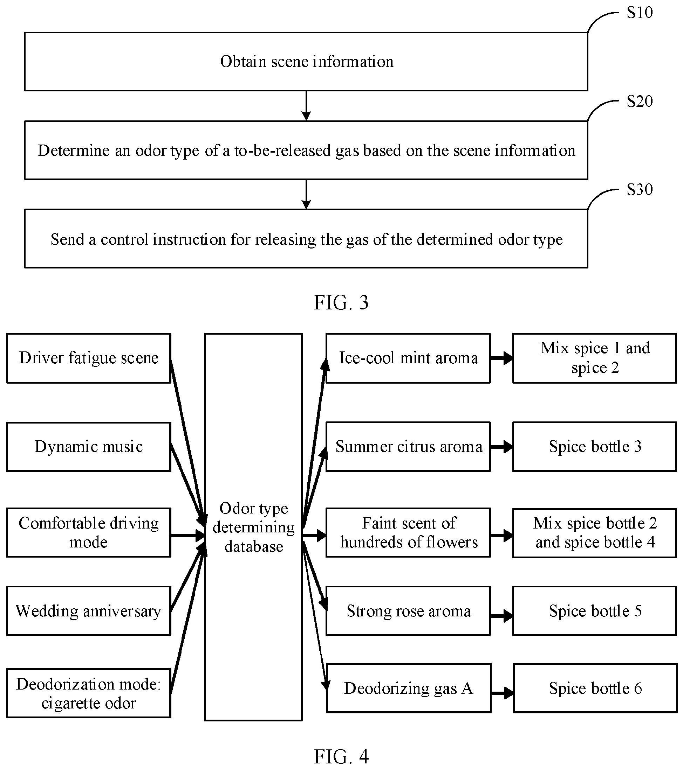

FIG. 3 is a schematic flowchart of a control method for an odor adjustment system 300 according to an embodiment. The control method is performed by a control apparatus 200. As shown in FIG. 3, the control method includes the following: S10: Obtain scene information. S20: Determine a target odor type of a to-be-released gas based on the scene information. S30: Send a control instruction for releasing the gas of the target odor type. That is, the control instruction includes a gas release instruction for releasing the gas of the determined target odor type.

For example, when the odor adjustment apparatus 100 is disposed on a vehicle, the scene information in S10 includes schedule reminder information and one or more of driving fatigue scene information (driver status information), user emotion information, off-odor removal scene information, driving mode information, music scene information, environment information, historical scene record information, or user-defined scene information.

The driving fatigue scene information indicates a fatigue status of a driver. The driving fatigue scene information may be obtained based on captured image information of a camera and/or detection information of a grip sensor (a pressure sensor) on a steering wheel.

The user emotion information is information that can indicate a user emotion status. Image recognition may be performed on image information captured by a camera to obtain a facial expression of a user, to determine an emotion status (for example, an anger state) of the user, to obtain the user emotion information. In addition, the emotion status of the user may be further determined based on audio information detected by a microphone. Specifically, for example, a user emotion may be determined based on a speech speed, a tone, a volume, or the like, or a user emotion may be determined based on some specific keywords included in a voice. In addition, the user emotion information may be further determined based on operation action information, and the operation action information includes, for example, an operation of frequently honking a vehicle horn.

The off-odor removal (which may be referred to as deodorization for short) scene information is information, for example, location information (for example, near a hot pot store), consumption information (for example, a payment record in a hot pot store) of the user, or a voice instruction of the user, for example, "help me remove the smoke odor", that can indicate that a current scene is a scene in which odor removal is required. In addition, whether off-odor removal is required may be further determined based on detection information of an odor sensor (for example, a sensor that can detect a cigarette odor).

In this embodiment, in S30, when a deodorization mode enabling condition is met (a condition indicated by the off-odor removal scene information is met), a control instruction is sent, where the control instruction includes a gas release instruction for releasing a deodorizing gas. The deodorization mode enabling condition herein includes one or more of the following: receiving a deodorization instruction of the user, receiving deodorization confirmation information of the user, detecting an off-odor, detecting a deodorization requirement, and detecting a first user action.

The deodorization instruction of the user includes one or more of the following: a voice deodorization instruction of the user, a gesture deodorization instruction, and a deodorization instruction generated by the user through a button (a physical button) or a selection operation on a touchscreen. The voice deodorization instruction is a voice instruction sent by the user through a microphone, for example, "please help me remove the smoke odor". The gesture deodorization instruction refers to a gesture motion (a mid-air operation) performed by the user, and the gesture motion represents an instruction "please help me remove the smoke odor".

The off-odor herein includes one or more of the following detected by a smoke sensor or an off-odor sensor: a cigarette odor, an oil smoke odor, a hot pot odor, a barbecue odor, and an air conditioner off-odor. In addition, the air conditioner off-odor may be obtained based on information sent by a controller of an air conditioner.

The detected deodorization requirement includes a deodorization requirement predicted based on user information, where the user information includes one or more of the following: consumption information and location information.

The deodorization confirmation information of the user includes voice confirmation information, gesture confirmation information, or confirmation information generated through a button or a selection operation on the touchscreen that is fed back by the user after a deodorization request is sent to the user.

The first user action includes one or more of the following: a nose covering action, a fanning action, a clothes shaking action, and a window opening action.

Herein, off-odor removal refers to releasing a deodorizing gas, to eliminate or suppress an off-odor in a manner like shielding, counteracting, decomposing, or adsorbing. The deodorizing gas is a gas with these deodorization functions. The deodorizing gas may be the same as a released gas in another scene, or a spice for the deodorizing gas may be separately disposed, and there may be one or more spices.

In an embodiment, before performing an operation of releasing a deodorizing gas, a control apparatus may send a query message to a user, so that the user confirms whether to perform the operation (for example, an option is provided on a man-machine interaction interface, or a query is sent by using a voice, and the user may confirm by using the voice). The user may choose not to change an aroma of the user, or may choose to accept changing an aroma of the user.

In addition, an aroma enhancement function may be provided for the user, that is, an option is provided on the man-machine interaction interface, or a voice instruction of the user is received, to release an aroma gas, to achieve an effect similar to "spraying perfume", so that the user has an aroma on the body (on clothing). In this way, the user may increase an aroma of the user by taking a car or at home.

In addition, in an off-odor removal mode, the control apparatus may detect a location of the user, and control the odor adjustment apparatus to blow a deodorizing gas towards the location of the user. It may be understood that, the odor adjustment apparatus has a structure that can change a direction of an exhaust vent. Specifically, for example, an exhaust vent is disposed on a side wall of a housing of the odor adjustment apparatus, and a housing part on which the exhaust vent is disposed may be driven to rotate relative to another housing part. In addition, in another implementation, the odor adjustment apparatus may be disposed in an air exhaust channel of an air conditioner, and a direction of an air flow that is discharged is changed by adjusting a direction of a deflector of an air outlet of the air conditioner, to blow a gas towards the user. The user herein is, for example, a driver, and a location of the user may be obtained in a manner of sound positioning based on a sound emitted by the driver, or may be obtained by using an image taken by a camera.

The schedule reminder information is, for example, reminder information recorded by a calendar application, and is used to remind the user of a to-do item at a specific time. For example, the user records, in a calendar application, a specific day of an anniversary (including a wedding anniversary, a birthday, and the like), or records a day "go to the playground with the child at 14:00". It can be learned from the above that the schedule reminder information includes reminder content and a reminder time, the reminder content indicates reminder content entered by the user, and the reminder time indicates a reminder time selected by the user (which may be only a date, namely, a day, or may be a moment that is accurate to hours, minutes, and seconds). For example, to remind the user of an event "May 15 is a wedding anniversary", the user operates a calendar application, and enters a text "Wedding anniversary" in a reminder content column of May 15. In this case, the reminder content is "Wedding anniversary", and a reminder time is May 15.

It should be noted that the reminder content does not necessarily include specific content of a to-do list. For example, for an anniversary, the user records only what an anniversary is, but does not necessarily record what needs to be done on the anniversary.

In addition, the schedule reminder information may be obtained from a record of an in-vehicle calendar application, or may be obtained from a record of a calendar application of a mobile phone of the driver. In this case, the control apparatus 200 can directly or indirectly communicate with the mobile phone of the driver. In addition, when the odor adjustment apparatus is configured indoors, the schedule reminder information may be obtained from a record of a calendar application of a mobile phone of the user.

In addition, in addition to the calendar application, the schedule reminder information may alternatively be obtained from another program. For example, when the user records information "May 15 is a wedding anniversary" in a memo application or a to-do list application, the control apparatus 200 may also determine, based on the information, an odor type of a to-be-released gas, and enable the odor adjustment apparatus 100 to release an aroma gas of a corresponding odor type. In addition, some chat programs may also include a schedule reminder function, and in this case, the schedule reminder information may also be obtained from the chat programs. The driving mode information indicates a current driving mode of the vehicle, and the driving mode is set by the user (driver), for example, includes a sport mode or a comfort mode.

The music scene information indicates a music type, for example, dynamic music, joy music, national music, and soft music. The music scene information may be obtained from an in-vehicle infotainment system. In an embodiment, the control apparatus 200 may also be a control apparatus of the in-vehicle infotainment system. In this way, music information may be easily obtained. In addition, in another embodiment, the control apparatus 200 may also be disposed independently of the in-vehicle infotainment system.

The environment information includes, for example, temperature information and/or humidity information, and may be obtained by using a detection signal of a temperature sensor or a humidity sensor. In addition, the scene information may further include in-vehicle atmosphere light information, and the like.

The historical scene record information is a historical operation record of the user about releasing an aroma gas. For example, the user performs an active operation at a specific place (a tourist attraction, a school, a theater, or the like) to enable the odor adjustment apparatus to release an aroma gas of a specific odor type (a pure odor type or a mixed odor type). In this case, the control apparatus stores the operation of the user in a historical scene database in association with the place. Then, when it is determined, based on location information of the vehicle, that the place is reached, the aroma gas of the foregoing odor type is automatically released based on a record in the historical scene database. In addition to the place, the same applies to a time point and people (when with someone). For example, the user usually enables the odor adjustment apparatus to release an aroma gas of a refreshing odor type at 20:00 p.m., or when a person takes a car with the user, the user often enables the odor adjustment apparatus to release an aroma gas of a refreshing odor type, and the control apparatus stores time point information, person information, and odor type information in an associated manner in a historical scene database, and then automatically releases an aroma gas of a corresponding odor type when a condition indicated by the time point information or the person information is met.

It may be understood that, to enable the user to perform an active operation, the odor adjustment system should have a corresponding operation function. For example, a corresponding operation button is provided on the man-machine interaction interface of a central display screen of the vehicle, including an on/off button, an odor type setting button, and the like. These operation buttons correspond to function modules of the control apparatus.

The user-defined scene information is scene information defined by the user. For example, an odor type setting button, a release time setting button, and a release place setting button are provided on the man-machine interaction interface of the central display screen of the vehicle. By performing operations on these buttons, the user can enable the odor adjustment apparatus to automatically release one or more aroma gases of some odor type or several odor types at a time (a time point or a time period) and/or a place.

It may be understood that the odor type setting button, the release time setting button, and the release place setting button correspond to an odor type setting function module, a release time setting function module, and a release place function module of the control apparatus. When the user taps these buttons, an odor type setting interface, a release time setting interface, and a release place setting interface are correspondingly displayed on the man-machine interaction interface.

In addition, in terms of odor type setting, the user taps the odor type setting button to display the odor type setting interface, and the odor type setting interface may provide the user with a function of editing an odor type spectrum (corresponding to a function that is provided by the control apparatus and that enables the user to edit the odor type spectrum). The so-called odor type spectrum herein means that an odor type changes with time, and specifically, a diffusing rate of various spices in a spice accommodating mechanism changes with time. If the diffusing rate is represented by percentage, the diffusing rate of various spices may be controlled to change between 0% and 100%, where 0 indicates that the spices are not diffused, and 100% indicates that the spice diffusing mechanism diffuses the spices with maximum power. This control may be implemented, for example, through so-called duty cycle control. By controlling the diffusing rate of various spices, concentration control of an aroma gas, control of an odor type, release duration control of an aroma gas of a specific odor type, and release sequence control of aroma gases of a plurality of odor types can be implemented.

It is assumed that there are three types of spices: A, B, and C, and diffusing rates of two of the three types of spices are 0, and a diffusing rate of the other type of spice is not 0. In this way, a specific type of spice can be separately diffused. For example, diffusing rates of the spices A and B are 0, and a diffusing rate of the spice C is 100%, and the spice C can be separately diffused. In this case, concentration of an aroma gas formed by the spice C may be further adjusted by adjusting the diffusing rate of the spice C. In addition, if diffusing rates of more than two spices are not 0, mixing and emission may be implemented. For example, if the diffusing rates of the spices A and B are 100%, and the diffusing rate of the spices C is 0, mixedly diffusing of the spices A and B may be implemented, to obtain an odor type formed by mixing the two spices. If the diffusing rate of the spice B is adjusted to 50%, another odor type formed by mixing the two spices is obtained. In addition, after the spice A is distributed for a specific period of time, the spice B may be diffused (in this case, the spice A stop being diffused or continue to be diffused).

In addition, for example, when the odor adjustment apparatus 100 is disposed in a home room, the scene information includes calendar reminder information and one or more of user emotion information, off-odor removal scene information, music scene information, environment information, indoor light information, historical scene information, or user-defined scene information.

In S20, the odor type of the to-be-released gas is determined based on the obtained scene information. A correspondence between scene information and an odor type may be prestored in a database, and the odor type of the to-be-released gas is determined based on the correspondence. FIG. 4 shows a control method for determining an odor type based on scene information according to an embodiment. As shown in FIG. 4, when it is determined, based on driving fatigue scene information, that a driver is fatigued, it is determined to release a gas whose odor type is an ice-cool mint aroma; when it is determined, based on music scene information, that dynamic music is currently played, it is determined to release a gas whose odor type is a summer citrus aroma; when it is determined, based on driving mode information, that a vehicle is in a comfortable driving mode, it is determined to release a gas whose odor type is a faint scent of hundreds of flowers; when it is determined, based on schedule reminder information, that today is a wedding anniversary, it is determined to release a gas whose odor type is a strong rose aroma; and when it is determined, based on off-odor removal scene information, that a cigarette odor needs to be removed, it is determined to release a deodorizing gas whose type is A.

For another example, when it is detected, based on user emotion information, that a user is anger, an aroma gas that can relieve an emotion is released; and when it is determined, based on off-odor removal scene information, that off-odor removal is required, an aroma gas that can remove (cancel) an off-odor (for example, a hot pot odor or a cigarette odor) on the user (on clothes) or an off-odor (for example, a smoke odor or a cigarette odor) in an environmental space is released, an aroma of the aroma gas may be determined based on detection information of an odor sensor, or may be determined based on location information, consumption information of a user, or according to a voice instruction of a user. For example, when the local location information indicates that the vehicle is located near a hot pot shop, an odor type that can remove a hot pot odor is determined, or a gas of a default odor type can be released.

In addition, gases of these odor types may be released at the same time. For example, when it is detected that a driver is fatigued and today is a wedding anniversary, a gas of an ice-cool mint aroma and a gas of a strong rose aroma may be released at the same time.

The following further describes processing of determining, based on schedule reminder information, a target odor type of a to-be-released gas.

When obtaining the schedule reminder information, the control apparatus 200 may perform semantic understanding (which may also be referred to as natural language understanding (Natural Language Understanding, NLU)) processing on reminder content information (text reminder information) in the schedule reminder information, to learn a meaning of the reminder content information. On this basis, an odor type of a to-be-released gas is determined based on the text reminder information. For example, when reminder content indicated by the schedule reminder information is "wedding anniversary", it is determined that an odor type of a to-be-released gas is a rose aroma.

In addition, in addition to text reminder information, a specific form of reminder content information further includes voice reminder information. In this case, the control apparatus 200 may learn a meaning of the voice reminder information through voice recognition (also referred to as automatic speech recognition (Automatic Speech Recognition, ASR)). On this basis, another to-be-released odor type is determined based on the voice reminder information.

In addition, it may be understood that the reminder time information may indicate a day, or may indicate a moment more specifically.

The target spice type determined in S20 may be an odor type formed by one type of spice in the spice accommodating mechanism, or may be an odor type formed by a plurality of types of spices.

In S30, based on the target odor type that is of the to-be-released gas and that is determined in S20, a control instruction for releasing a gas of the determined odor type is generated and sent. For example, when driving fatigue scene information indicates that a driver is fatigued, it is determined that another to-be-released odor type is a mint aroma. In this case, in S30, a control instruction for releasing a mint aroma gas is generated, and the control instruction is sent to the odor adjustment apparatus 100, so that the odor adjustment apparatus 100 releases the mint aroma gas to an environmental space.

In addition, for example, when the odor type of the to-be-released gas is determined based on the schedule reminder information, in S30, the control instruction for releasing the gas of the determined odor type is generated and sent. For example, when reminder content information is "wedding anniversary", a control instruction for releasing a gas with a strong rose aroma is sent.

In addition, optionally, in S30, current time information may be obtained, and when the current time information matches the reminder time information in the schedule reminder information, the foregoing control instruction is generated and sent. For example, when the reminder content information is "go to the playground with the child at 14:00", the current time information is obtained, and the control instruction is sent when the current information is close to 14:00, arrives at 14:00, or slightly exceeds 14:00.

In another embodiment, it may alternatively be determined in S20 whether the current time information matches the reminder time information, and when the current time information matches the reminder time information, the processing of determining an odor type based on the schedule reminder information described above is performed, and when the current time information does not match the reminder time information, the processing of determining an odor type based on the schedule reminder information is not performed. In this way, processing energy consumption may be reduced.

In addition, optionally, in S30, a target person may be determined based on the reminder content information, and when the target person is detected, the foregoing control instruction is sent. For example, when the reminder content information is "wedding anniversary", it is determined that the target person is a spouse of a user such as a driver or a mobile phone owner; and when the reminder content information is "go to the playground with the child at 14:00", it is determined that the target person is a child of a user such as a driver or a mobile phone owner. In addition, a target person condition herein may be combined with a time condition.

Herein, detection of the target person may be implemented by performing person recognition through image information. For example, when the odor adjustment apparatus is disposed on a vehicle, for example, a camera on the vehicle is used to take an image in the vehicle to obtain an in-vehicle image, and facial recognition processing is performed on the in-vehicle image, so that whether the target person exists in the vehicle can be determined. When the odor adjustment apparatus is disposed in a room at home, the room may be photographed by using a camera disposed in the room, to obtain an indoor image, and facial recognition processing is performed on the indoor image, so that whether the target person exists in the room can be determined. In addition, the target person may alternatively be detected by using audio information, that is, sound information is detected by using a microphone, and voiceprint recognition is performed on the detected sound information, to determine whether the target person exists in a vehicle or a room.

It may be understood that, to determine whether the target person is detected, the target person information, for example, facial information of the target person and voiceprint information of the target person may be prestored. As an example, a driver may separately establish corresponding accounts for a spouse and a child of the driver in a head unit system, and facial information and voiceprint information of the spouse and the child of the driver may be collected and stored when the accounts are established.

In addition, in an embodiment, the control apparatus may detect a location of a user or a target person, and control the odor adjustment apparatus to blow an aroma gas towards the location of the user or the target person.

In addition, in the embodiment shown in FIG. 4, the spice accommodating unit 110 includes a plurality of spice bottles that can be independently disassembled, and the plurality of spice bottles each accommodate one type of spice, and a target aroma gas may be generated by using a spice in a single spice bottle, or a target aroma gas may be generated by mixing gases formed by the spices in the plurality of spice bottles. In addition, the spice in a spice bottle may be a simple spice (for example, a mint spice), or may be a spice formed by mixing a plurality of simple spices.

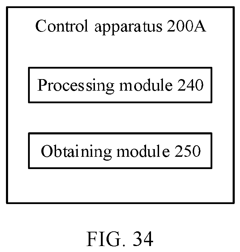

FIG. 34 is a block diagram of a structure of a control apparatus according to an embodiment of this application. The control apparatus 200A includes a processing module 240 and an obtaining module 250. The obtaining module 250 may perform various processing in S10, and the processing module 240 may perform various processing in S20 and S30. Specific content of the processing is described above, and details are not described herein again. The control apparatus 200A may implement functions of the processing module 240 and the obtaining module 250 by using a processor to execute a program (software) stored in the memory. In addition, the control apparatus 200A may also implement functions of the processing module 240 and the obtaining module 250 by using hardware such as an LSI (Large Scale Integration, large scale integrated circuit) and an ASIC (Application Specific Integrated Circuit, application-specific integrated circuit), or may implement functions of the processing module 240 and the obtaining module 250 by using a combination of software and hardware. The processing module 240 is a processor, and the obtaining module 250 is an interface circuit.

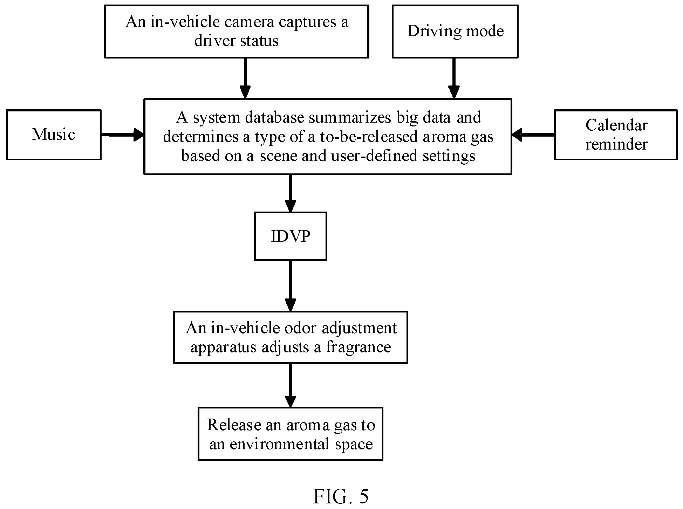

FIG. 5 is a schematic illustration diagram of implementation logic of an odor adjustment system according to an embodiment. Refer to FIG. 5. The odor adjustment system is an in-vehicle odor adjustment system, and an odor adjustment apparatus of the odor adjustment system is portable. When installed on a vehicle, the odor adjustment apparatus is connected to a control apparatus by using an intelligent vehicle digital platform (Intelligent digital vehicle platform, IDVP) interface. The control apparatus is, for example, integrated in a cockpit domain controller (Cockpit Domain Controller, CDC).

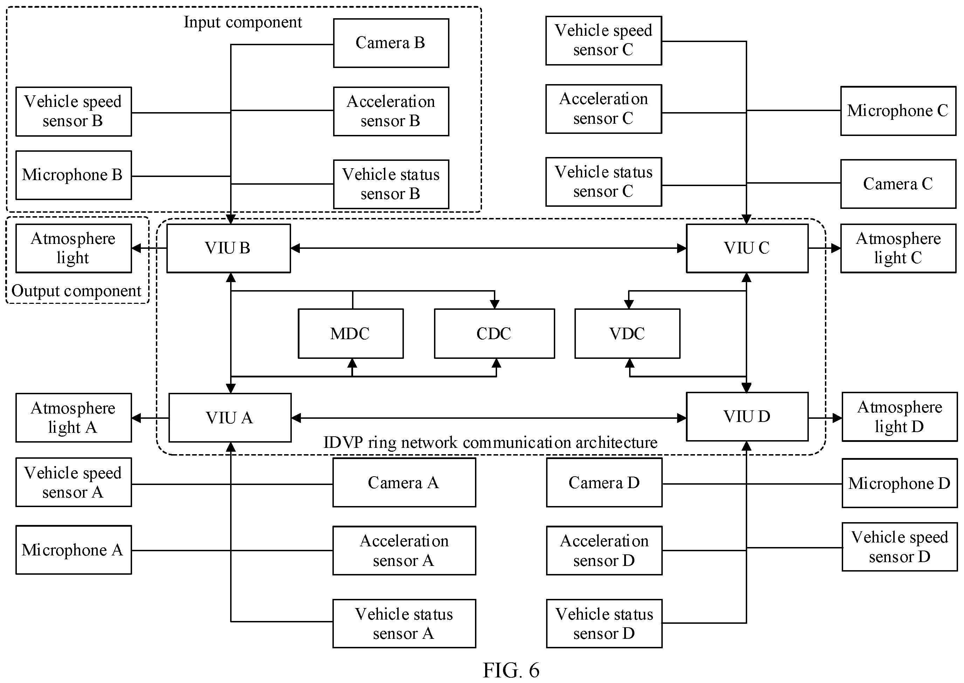

FIG. 6 is a schematic illustration diagram of a network architecture of an intelligent digital vehicle platform according to an embodiment. As shown in FIG. 6, in the architecture, there is a ring network formed by a plurality of vehicle integration units (Vehicle Integration Unit, VIU). The vehicle integration units are connected to input components such as a camera, an acceleration sensor, a vehicle status sensor, a vehicle speed sensor, and a microphone, and is further connected to output components such as an atmosphere light. Herein, the input components and the output components are merely examples, and may further include another input component (for example, a grip sensor) and an output component (for example, an odor adjustment apparatus). In addition, the cockpit domain controller, the mobile data center (Mobile Data Center, MDC), and the vehicle domain controller (Vehicle Domain Controller, VDC) are connected to the ring network, to receive input information of an input component and send output information to an output component through the ring network. In addition, the cockpit domain controller is configured to provide services for vehicle parts in the cockpit domain, where the vehicle parts in the cockpit domain include a head-up display, an instrument display, a radio, navigation, a camera, and the like. The mobile data center functions as an intelligent driving domain controller. The vehicle domain controller is configured to provide a service for a vehicle part in a vehicle body domain and a vehicle part in a chassis domain. The vehicle part in the vehicle body domain includes a door/window lifting controller, an electric rearview mirror, an air conditioner, a central door lock, and the like. The vehicle part in the chassis domain includes a vehicle part in a braking system, a vehicle part in a steering system, and a vehicle part such as a throttle in an acceleration system.

In addition, in an embodiment, the odor adjustment apparatus may further include an image playing mechanism. When an aroma gas is released, the image playing mechanism may project the released aroma gas (aerosol) to form an image that uses the aroma gas (aerosol) as a carrier. The image playing mechanism may be a holographic projection mechanism that can generate a holographic image. Correspondingly, in an embodiment of the control method, in S30, in addition to the gas release instruction, an image playing instruction may be further sent, so that the image playing mechanism plays an image that uses a released aroma gas as a carrier. In addition, specific content of the image may be determined based on a type of the aroma gas. Specifically, a correspondence between the type of the aroma gas and the image content may be pre-stored in a database. For example, a rose flavor corresponds to playing a rose image. In addition, in another embodiment, a default image may be further played, and the default image is irrelevant to the type of the aroma gas. In addition, an accommodating unit that stores water may be further disposed in the odor adjusting apparatus. When the foregoing image is played, water mist may be generated, to enhance aerosol concentration and improve image display quality.

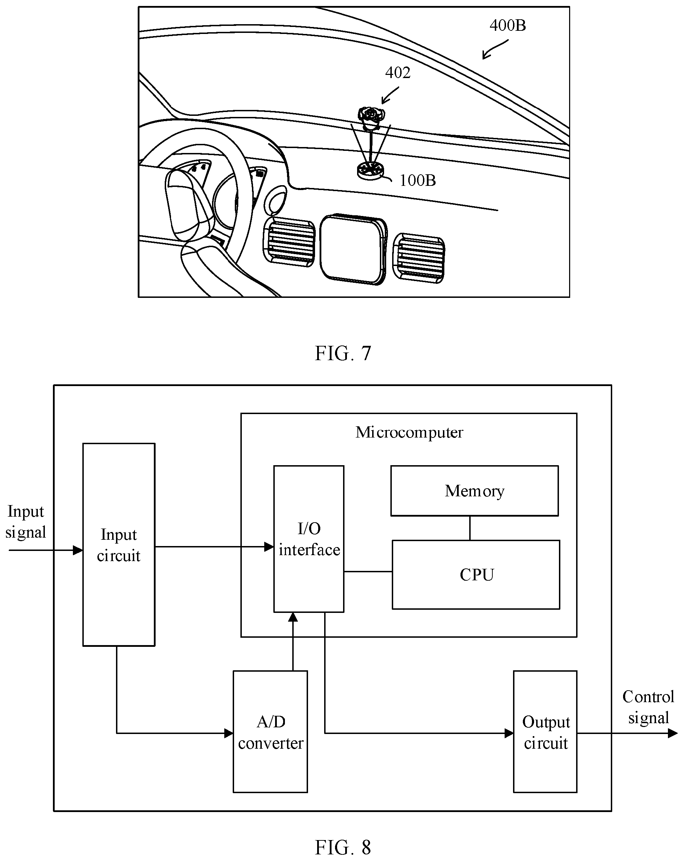

FIG. 7 is a schematic illustration diagram of an application scenario according to an embodiment of this application. As shown in FIG. 7, an odor adjustment apparatus 100B is disposed in a compartment of a vehicle 400B. The odor adjustment apparatus 100B can release an aroma gas to the compartment, and can display an image 402 that uses the aroma as a carrier.

As described above, the control apparatus may be implemented by an ECU. Optionally, one ECU may be used, or a plurality of ECUs may be used.

The ECU is a control apparatus including integrated circuits and configured to implement a series of functions such as data analysis, processing, and sending. As shown in FIG. 8, an embodiment of this application provides an electronic control unit ECU. The ECU includes a microcomputer (microcomputer), an input circuit, an output circuit, and an analog-to-digital (analog-to-digital, A/D) converter.

A main function of the input circuit is to preprocess an input signal (for example, a signal from a sensor), and a processing method varies depending on the input signal. Specifically, because there are two types of input signals: an analog signal and a digital signal, the input circuit may include an input circuit for processing an analog signal and an input circuit for processing a digital signal.

A main function of the A/D converter is to convert an analog signal into a digital signal. After being preprocessed by the corresponding input circuit, the analog signal is input to the A/D converter for processing, so that the analog signal is converted into a digital signal acceptable by the microcomputer
The output circuit is an apparatus for establishing contact between the microcomputer and an actuator. A function of the output circuit is to convert a processing result sent by the microcomputer into a control signal to drive the actuator to work. The output circuit generally uses a power transistor, and controls, according to an instruction of the microcomputer, an electronic circuit of an actuation element through turn-on or cutoff.

The microcomputer includes a central processing unit (central processing unit, CPU), a memory, and an input/output (input/output, I/O) interface. The CPU is connected to the memory and the I/O interface by using a bus, and information may be exchanged between the components by using the bus. The memory may be a memory such as a read-only memory (read-only memory, ROM) or a random access memory (random access memory, RAM). The I/O interface is a connection circuit for exchanging information between a central processing unit (central processor unit, CPU) and an input circuit, an output circuit, or an A/D converter. Specifically, the I/O interface may be classified into a bus interface and a communication interface. The memory stores a program, and the CPU may invoke the program in the memory to implement a function of the foregoing control apparatus, perform processing in S10 to S30, and perform the foregoing control method.

It can be learned from the foregoing that embodiments of this application further provide a computing device, a computer-readable storage medium, and a computer program that are configured to implement the foregoing control method.

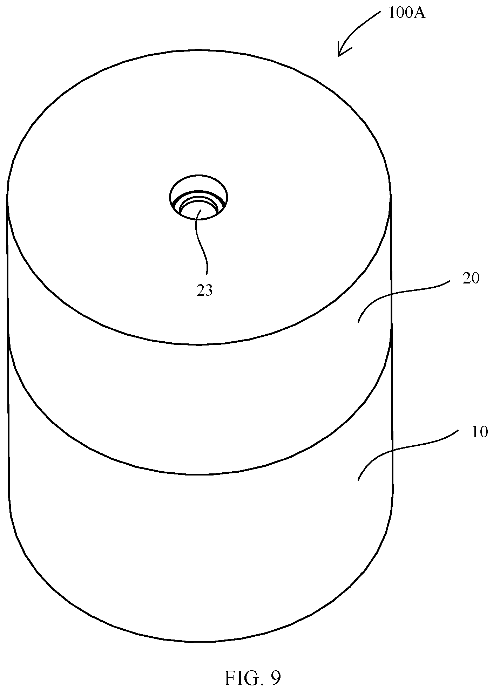

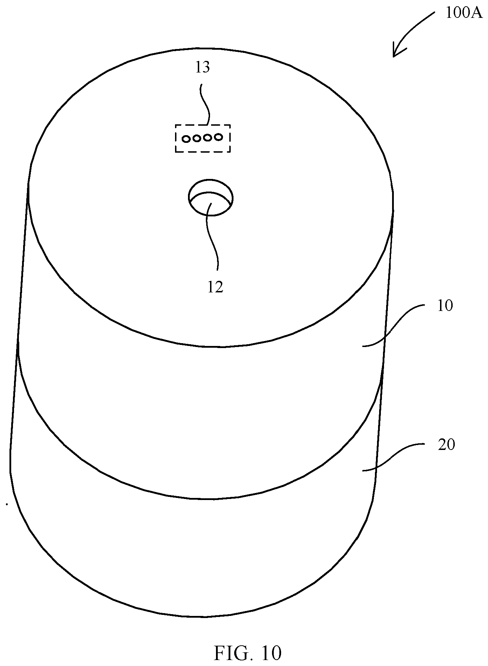

Refer to FIG. 9 to FIG. 26. The following describes an odor adjustment apparatus according to an embodiment of this application. FIG. 9 is a schematic diagram of a three-dimensional structure of an odor adjustment apparatus according to an embodiment of this application. FIG. 10 is a schematic diagram of another three-dimensional structure of an odor adjustment apparatus according to an embodiment of this application.

As shown in FIG. 9, the odor adjustment apparatus 100A includes a first housing 10 and a second housing 20, and the first housing 10 and the second housing 20 are assembled. In this embodiment, the first housing 10 and the second housing 20 are cylindrical in shape. However, in another implementation, the first housing 10 and the second housing 20 may alternatively be in another shape, for example, in a cuboid shape or a prism shape. A discharge hole 23 is disposed on the second housing 20, and an aroma gas is diffused to an environmental space through the discharge hole 23 (which is specifically described in the following).

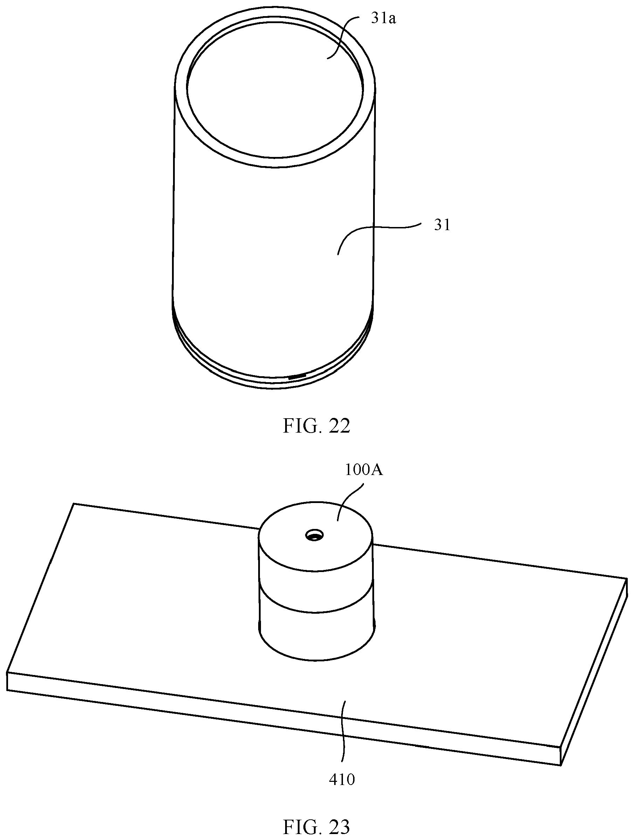

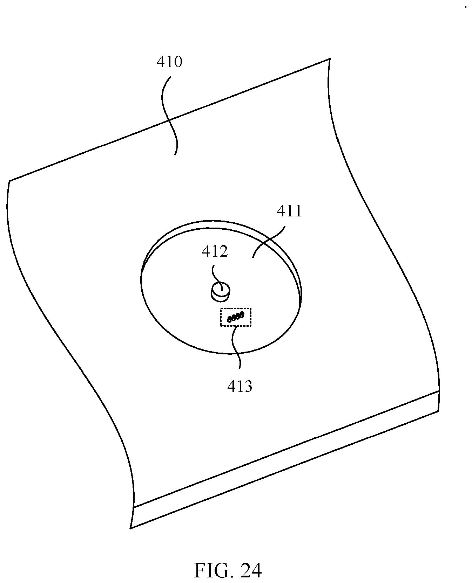

The odor adjustment apparatus 100A in this embodiment is portable, and may be configured in a plurality of environmental spaces, for example, configured in a vehicle or in an indoor area of a building. FIG. 23 and FIG. 24 show related structures in which an odor adjustment apparatus is disposed in a vehicle. As shown in FIG. 23, as a specific installation structure embodiment, the odor adjustment apparatus 100A may be installed on a window sill plate of a vehicle, and may be on a front window sill plate or a rear window sill plate. In this embodiment, an example in which the odor adjustment apparatus 100A is installed on a front window sill plate 410 is used for description. In addition, the figure merely schematically shows a part of the front window sill plate 410, and cannot be equivalent to an actual product.

The following briefly describes an installation structure of the odor adjustment apparatus 100A in this case. As shown in FIG. 10, a positioning hole 12 and a plurality of wiring holes 13 are disposed on the first housing 10. In addition, as shown in FIG. 24, a mounting groove 411 is disposed on the front window sill plate 410, the entire mounting groove 411 is in a circular countersunk hole shape, and a positioning protrusion 412 and a plurality of binding posts (pins) 413 are disposed on a bottom surface of the mounting groove 411. When the odor adjustment apparatus 100A is installed on the front window sill plate 410, the first housing 10 is inserted into the mounting groove 411. The positioning protrusion 412 is embedded in the positioning hole 12, and the plurality of binding posts 413 are respectively inserted into the plurality of wiring holes 13 to implement a corresponding electrical connection, so that the odor adjustment apparatus 100A is electrically connected to, for example, a cockpit domain controller (control apparatus), and is controlled by the cockpit domain controller. In addition, the odor adjustment apparatus can be powered by a power supply (battery) on a vehicle. The plurality of wiring holes 13 herein correspond to the communication interface and the power supply interface in this application.

In addition, in this embodiment, a magnet (not shown in the figure) is disposed at a location that is on an inner side of the first housing 10 and that corresponds to the positioning hole 12, and a magnet (not shown in the figure) is disposed inside the positioning protrusion 412 on the front window sill plate 410. When the positioning protrusion 412 is embedded with the positioning hole 12, magnets of the positioning protrusion 412 and the positioning hole 12 attract each other, so that the first housing 10 and the entire odor adjustment apparatus 100A can be in a stable installation state, for example, even when the vehicle collides, the odor adjustment apparatus 100A can still maintain the stable installation state. In addition, in an embodiment, the first housing 10 may be further connected to the front window sill plate 410 by using a threaded connection structure.

In addition, in the foregoing description, wiring holes 13 are disposed on the odor adjusting apparatus 100A, and a binding post 413 is disposed on a side of the front window sill plate 410 of the vehicle, to implement an electrical connection between the odor adjusting apparatus 100A and a vehicle body side apparatus (for example, a cockpit domain controller). However, this application is not limited thereto. In another embodiment, contact points may be separately disposed on the odor adjusting apparatus 100A and the front window sill plate 410, to implement an electrical connection between the odor adjustment apparatus 100A and the vehicle body side apparatus through contact between the contact points of the odor adjustment apparatus 100A and the vehicle body side apparatus.

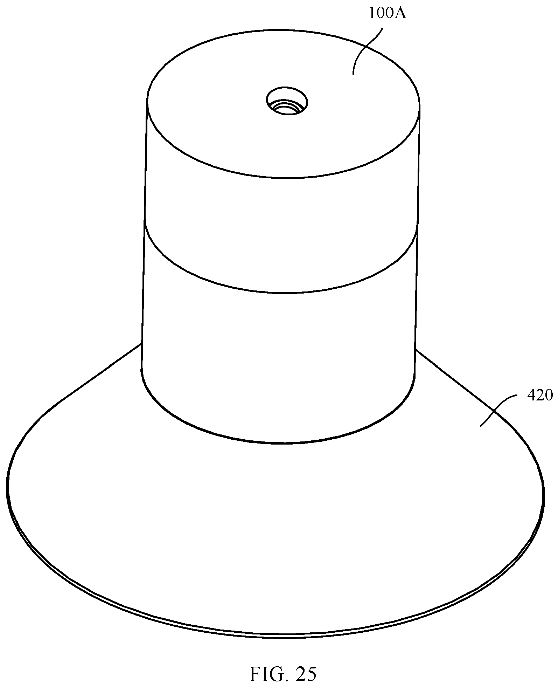

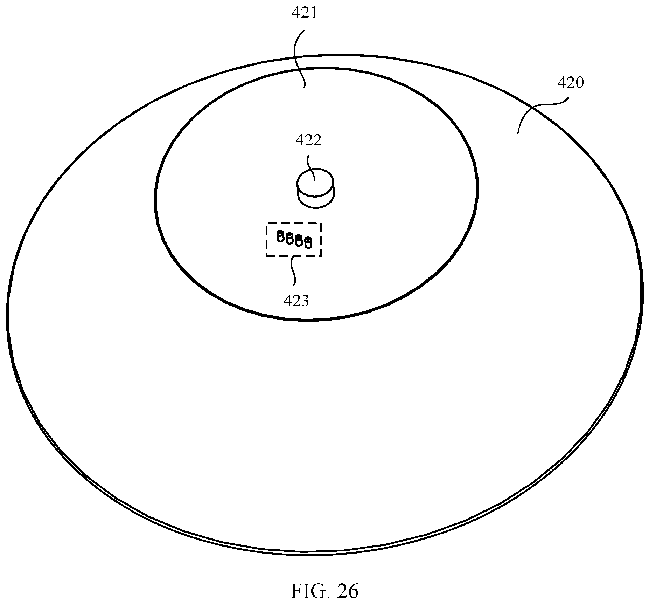

FIG. 25 and FIG. 26 show related structures of the odor adjustment apparatus 100A configured in the vehicle. As shown in FIG. 25, the odor adjustment apparatus 100A may be installed on a base 420, where the base 420 may be portable, and may further form a sound box. As shown in FIG. 26, the base 420 has a mounting groove 421, and a positioning protrusion 422 and a plurality of binding posts 423 are disposed on a bottom surface of the mounting groove 421. In an installation state of the odor adjustment apparatus 100A, the first housing 10 is inserted into the mounting groove 421. The positioning protrusion 422 is embedded in a positioning hole 12, and the plurality of binding posts 423 are respectively inserted into a plurality of wiring holes 13 to implement a corresponding electrical connection, so that the odor adjustment apparatus 100A can be controlled by the base 420 (specifically, a control apparatus in the base 420) and powered by the base 420.

The following describes a specific structure of the odor adjustment apparatus 100A.

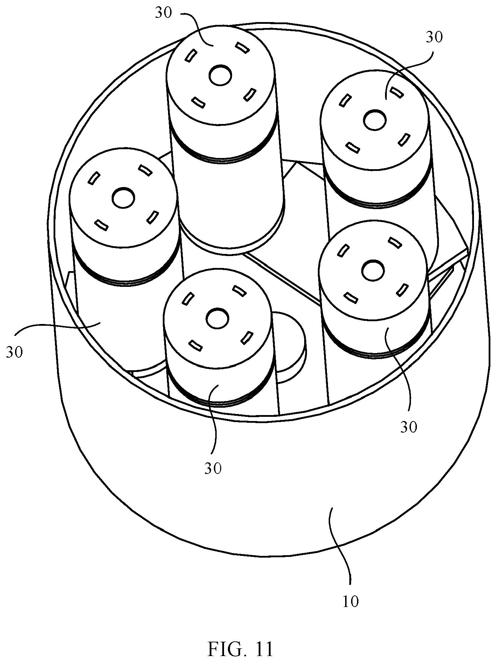

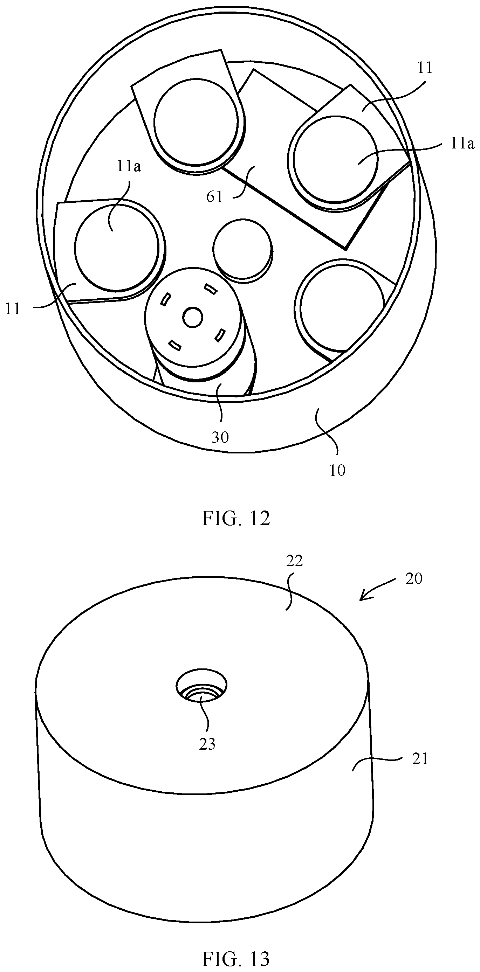

FIG. 11 is a schematic diagram of a three-dimensional structure of a first housing equipped with a spice container according to an embodiment of this application. FIG. 12 is a schematic diagram of another three-dimensional structure of a first housing according to an embodiment of this application.

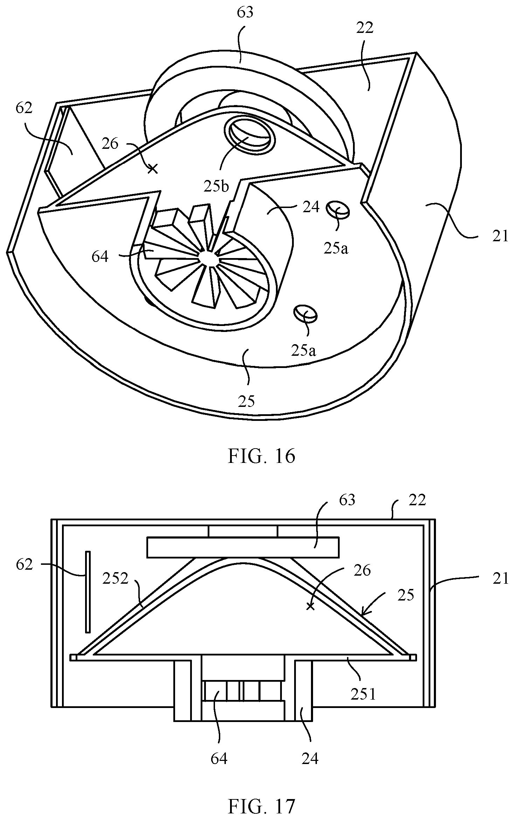

As shown in FIG. 11, a plurality of spice containers 30 are mounted in the first housing 10, and the plurality of spice containers 30 contain spices of different odor types. Under control of a control apparatus, the spices may be released independently, or may be released mixedly in any combination, so that aroma gases of odor types of which a quantity is larger than that of the spice containers 30 can be generated. As shown in FIG. 12, a plurality of support bases 11 are disposed in the first housing 10, and a support protrusion 11a is disposed on the support base 11. As shown in FIG. 22, a positioning concave part 31a is disposed on the first housing 10 of the spice containers 30. In a state in which the spice container 30 is installed in the first housing 10, the spice container 30 is supported by the support base 11, and the support protrusion 11a of the support base 11 is embedded in the positioning concave part 31a on the spice container 30, to keep the spice container 30 in a stable location. In addition, optionally, the spice container 30 may be clamped between the support base 11 and a bottom plate 251 (FIG. 17) of a mixing cavity housing 25 with a specific clamping force, so that a location of the spice container 30 can be maintained more stably. In addition, the plurality of spice containers 30 correspond to the spice accommodating mechanism in this application.

In addition, in this embodiment, the plurality of spice containers 30 are detachably mounted in the first housing 10. Therefore, when a spice in a spice container 30 is exhausted, the spice container 30 may be replaced with a new spice container 30. In another embodiment, a fixedly disposed spice container may also be used.

As shown in FIG. 12, a controller 61 (a circuit board) is configured in the first housing 10. The controller 61 is electrically connected to the wiring hole 13. When the odor adjustment apparatus is installed on a vehicle body or a sound box, the controller 61 is electrically connected to a control apparatus on a vehicle body side or the sound box side through the wiring hole 13, receives control instructions of the control apparatus, performs control according to the control instructions, and sends corresponding information in response to the control instructions.

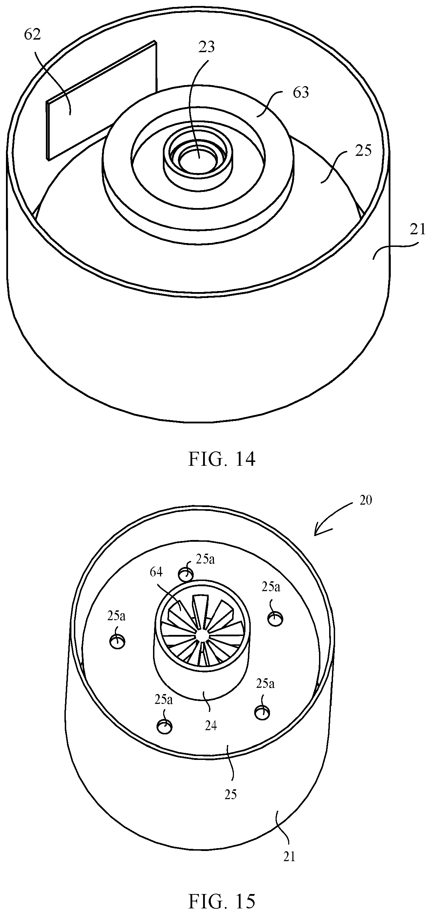

FIG. 13 is a schematic diagram of a three-dimensional structure of a second housing according to an embodiment of this application. FIG. 14 is a schematic diagram of another three-dimensional structure of a second housing according to an embodiment of this application. FIG. 15 is a schematic diagram of still another three-dimensional structure of a second housing according to an embodiment of this application. FIG. 16 is a schematic sectional view of a second housing according to an embodiment of this application. FIG. 17 is a schematic sectional view of a second housing according to an embodiment of this application.

As shown in FIG. 13, the second housing 20 has a main part 21 and a cover plate 22, and a discharge hole 23 is disposed on the cover plate 22. In this embodiment, the cover plate 22 is made of a transparent material or a translucent material. As shown in FIG. 14, a controller 62 (a circuit board) and a light emitting lamp 63 are disposed in the main part 21, and the controller 62 is electrically connected to the light emitting lamp 63. Under the control of the controller 62, the light emitting lamp 63 may emit light, and the light of the light emitting lamp 63 is emitted into an environmental space through the cover plate 22. In this embodiment, the entire light emitting lamp 63 is annular, and is disposed around the discharge hole 23 when being observed along an axis direction of the light emitting lamp 63. In addition, the light emitting lamp 63 is, for example, an LED lamp. In addition, the controller 62 is further electrically connected to the controller 61, and receives control instructions of the controller 61, performs control according to the control instructions of the controller 61, and sends corresponding information in response to the control instructions. The control instructions include, for example, a control instruction for enabling the light emitting lamp 63 to emit light and a control instruction for enabling the following fan 64 (FIG. 15) to start.

As shown in FIG. 14 to FIG. 16, a mixing cavity housing 25 is disposed in the second housing 20, the mixing cavity housing 25 forms a mixing cavity 26, and after the spices in the plurality of spice containers 30 are diffused to the mixing cavity 26, the spices are discharged to the environmental space through the discharge hole 23. Specifically, the mixing cavity housing 25 has a main part 252 and a bottom plate 251, a plurality of through holes 25a are disposed on the bottom plate 251, the plurality of through holes 25a are separately connected to a spice container 30, and a spice diffused by the spice container 30 enters the mixing cavity 26 through the through holes 25a. In addition, an opening 25b is disposed on the main part 252 of the mixing cavity housing 25, and the opening 25b communicates with the discharge hole 23, so that a spice gas in the mixing cavity 26 can be diffused to an environmental space through the opening 25b and the discharge hole 23.

In addition, a fan installation part 24 is fastened to the bottom plate 251 of the mixing cavity housing 25, and a fan 64 is installed on the fan installation part 24. The fan 64 is controlled by the controller 62, and a space where the fan 64 is located communicates with the mixing space 26, so that the fan 64 blows the aroma gas in the mixing space 26 to be diffused to the environmental space through the opening 25b and the discharge hole 23. The fan 64 corresponds to the blowing mechanism in this application.

By disposing the mixing cavity 26, spices diffused by any quantity of spice containers 30 in the plurality of spice containers 30 can be mixed in the mixing cavity 26, so that more aroma gases of different types can be adjusted.

In addition, in this embodiment, the main part 252 of the mixing cavity housing 25 is approximately in a cone shape, and the opening 25b is provided on a top of the main part 252, so that an aroma gas can be easily diffused.

In addition, the main part 252 of the mixing cavity housing 25 is formed into a cone shape, so that a space can be formed between the mixing cavity housing 25 and the main part 21 (and the cover plate 22), and the controller 62 and the light emitting lamp 63 are configured in the space, so that the odor adjustment apparatus 100A can be compact in structure.

The following describes a structure of the spice container in this embodiment.

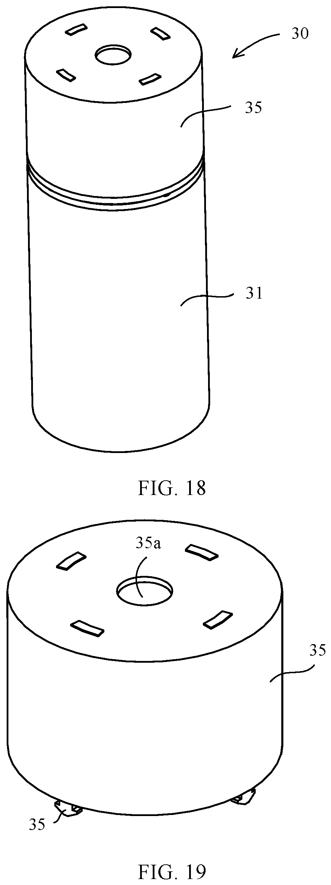

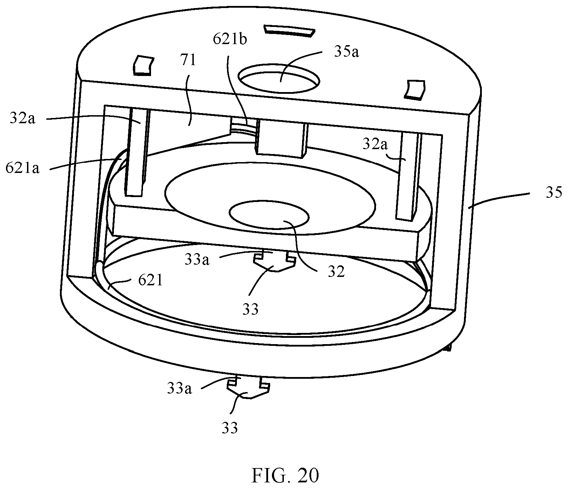

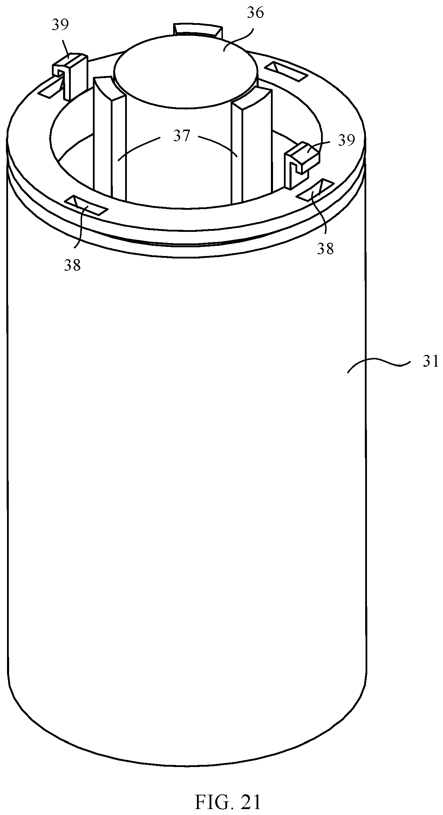

FIG. 18 is a schematic diagram of a three-dimensional structure of a spice container according to an embodiment of this application. FIG. 19 is a schematic diagram of a three-dimensional structure of a container cover of a spice container according to an embodiment of this application. FIG. 20 is a schematic sectional view of a container cover of a spice container according to an embodiment of this application. FIG. 21 is a schematic diagram of a three-dimensional structure of a container body of a spice container according to an embodiment of this application. FIG. 22 is a schematic diagram of another three-dimensional structure of a container body of a spice container according to an embodiment of this application.

As shown in FIG. 18, the spice container 30 is approximately cylindrical from an external perspective, and has a container body 31 and a container cover 35. As shown in FIG. 20, a controller 71 (a circuit board) is disposed in the container cover 35, where identification information is recorded, and the identification information includes odor type information indicating a spice in a spice container 30, so that a control apparatus (for example, a cockpit domain controller) can obtain an odor type of the spice in the spice container 30. In addition, the identification information may further include production place information, manufacturer information, production time information, or spice remaining amount information (remaining milliliters), and the like. The controller 71 is further configured to control an atomization component 32, so that the spice in the spice container 30 is diffused. The controller 71 may send the identification information actively or in response to a request of the control apparatus, and the control apparatus performs corresponding processing based on the identification information, which specifically includes: determining, based on the spice remaining amount information, whether a remaining amount of any spice is sufficient; and when a remaining amount indicated by the spice margin information is less than a remaining amount threshold, sending alarm information, so that a user knows that the spice is insufficient in a timely manner, thereby improving user experience. In addition, the control apparatus performs verification based on the production place information, the manufacturer information, the production time information, and the like, and sends alarm information when the information is inconsistent with the preset verification information or the information cannot be read, thereby avoiding incorrect use of a spice that does not match a specification and improving user experience. The production place information, the manufacturer information, or the production time information is an example of verification information. It may be understood that the verification information may alternatively be other information.

The container body 31 and the container cover 35 are installed together to form an accommodation space for accommodating a spice. When the spice in the spice container 30 is a liquid, the accommodation space may be a sealed space. In addition, in this embodiment, the container body 31 may be made of glass or plastic, and the container cover 35 may be made of plastic. In this embodiment, the container body 31 and the container cover 35 are installed together by using a clamping structure. Specifically, as shown in FIG. 21, a clamping hole 38 is fixedly disposed on the container body 31. In addition, as shown in FIG. 20, a clamping jaw 33 is fixedly disposed on the container cover 35. The clamping jaw 33 is clamped into the clamping hole 38, so that the container cover 35 can be mounted on the container body 31. In another embodiment, a clamping jaw may alternatively be disposed on the container body 31, and a clamping hole may be disposed on the container cover 35.

In addition, in this embodiment, the clamping jaw 33 is connected to the container cover 35 through a fragile part 33a, and strength of the fragile part 33a is low. When the container body 31 in an installation state is separated from the container cover 35, the fragile part 33a breaks, so that the clamping jaw 33 remains in a state of clamping with the clamping hole 38 and is separated from the container body 31. In this way, after the container cover 35 is removed from the container body 31, the container cover 35 cannot be installed on the container body 31. Therefore, for example, the spice in the spice container 30 can be prevented from being maliciously replaced, thereby ensuring security.

The following describes another implementation structure for preventing the spice from being maliciously replaced. To enable the fragile part 33a to be reliably broken, strength of the fragile part 33a may be reduced as much as possible, for example, a material amount of the fragile part 33a is reduced, a thickness of the fragile part 33a is reduced, or the fragile part 33a is made of a material with a strength lower than that of a part clamped with the clamping hole 38.

As shown in FIG. 20, the controller 71 is disposed in the container cover 35, a safety wire 621 is connected to the controller 71, two ends of the safety wire 621 are electrically connected to the controller 71, and a ring part is in the middle. In addition, as shown in FIG. 21, a plurality of hooks 39 are disposed on the container body 31. In a state in which the container cover 35 is installed on the container body 31, the wire 621 (the ring part) is hooked by the hooks 39. When the container body 31 in the installation state is separated from the container cover 35, the safety wire 621 is pulled off by the hook 39. In this way, the electrical connection between the controller 71 and the safety wire 621 fails, and the controller 71 can detect that the container cover 35 is opened or a corresponding function of the controller 71 is damaged, and cannot perform a corresponding control function, for example, cannot receive a control instruction of a control apparatus (for example, a cockpit domain sensor) on a vehicle body side, and cannot respond to an instruction of the control apparatus to send information.

The following describes a structure for diffusing a spice to the outside of the spice container 30.

As shown in FIG. 21, an adsorption component 36 is disposed in the container body 31, one end of the adsorption component 36 is connected to a bottom surface of the container body 31, and the other end of the adsorption component 36 extends out of the container body 31. The adsorption component 36 can adsorb a liquid spice in the container body 31, and the spice reach the other end of the adsorption component 36 due to a capillary effect or the like. The adsorption component 36 may be, for example, a cotton swab.

In addition, as shown in FIG. 20, the controller 71 and the atomization component 32 are disposed in the container cover 35. In addition, a plurality of cables 32a are further disposed in the container cover 35. The cables 32a are electrically connected on the controller 71 (a specific electrical connection structure is not shown), and the cables 32a penetrate through the container cover 35 to form contacts on an outer surface of the container cover 35, so that the cables 32a can be electrically connected to a component (contact) outside the container cover 35, and the controller 71 is electrically connected to the controller 61, receives a control instruction of the controller 61, performs control according to the control instruction, responds to the control instruction of the controller 61, and sends information. The atomization component 32 is in a sheet shape, and the end that extends out of the container body 31 of the adsorption component 36 is in contact with the atomization component 32. The atomization component 32 is electrically connected to the controller 71, and can be powered and controlled by the controller 71. Under the control of the controller 71, the atomization component 32 generates vibration, and atomizes a spice on the adsorption component 36, so that the spice can be diffused outside the spice container 30. A specific structure of the atomization component 32 may be, for example, a thin film with stainless steel, where micron-level through holes are disposed on the thin film, ceramic electrodes are disposed on two sides of the thin film, and the ceramic electrodes are supplied with power, so that the stainless steel thin film vibrates, thereby atomizing the spice.

A through hole 35a is disposed on the container cover 35, and the atomized spice is discharged outward through the through hole 35a. In an installation state, the through hole 35a communicates with the through hole 25a (FIG. 15), so that the atomized spice is diffused to the mixing cavity 26 through the through hole 35a and the through hole 25a. A plurality of adsorption components 36, a plurality of atomization components 32, and a controller 71 that are mounted on a plurality of spice containers 30, a controller 61, a mixing cavity 26, and the like correspond to the spice diffusing mechanism in this application as a whole. An adsorption component 36, an atomization component 32, and a controller 71 on a single spice container 30 correspond to the spice diffusing mechanism in this application (that is, the spice diffusing mechanism includes the controller 61, the mixing cavity 26, and the plurality of spice distributing units).

In addition, in this embodiment, as shown in FIG. 21, a liquid level sensor 37 is disposed in the container body 31, and the liquid level sensor 37 is configured to detect a remaining amount of a liquid (spice) in the container body 31. The liquid level sensor 37 is electrically connected to the controller 71 (FIG. 20), and sends detection information to the controller 71. The controller 71 sends the received detection information to a control apparatus (for example, a cockpit domain controller) on a vehicle body side by using the controller 61.

In the foregoing description, the control apparatus is integrated into the cockpit domain controller on the vehicle body side. However, in another embodiment, the controller may alternatively be configured in a housing of the odor adjustment apparatus, for example, integrated into the foregoing controller 61. In this case, the controller 61 may obtain information (for example, scene information) from the cockpit domain controller on the vehicle body side. In addition, in this case, the controller 61 may determine whether a current environment is "vehicle", "home", or the like, to invoke different functions. The determining may be implemented by obtaining information from a cockpit domain controller on a vehicle body side or a control apparatus on a sound box side. In addition, in this case, the odor adjustment apparatus may be powered by a power supply on a vehicle body side through a power supply interface, or a power supply (battery) may be configured on the odor adjustment apparatus.

The following summarizes relationships between the controllers described above. The controller 61 is a controller of the entire odor adjusting apparatus, the controller 62 is configured to control a light emitting lamp, the controller 71 is a controller on a spice container, and is configured to control an atomization component, the controller 62 and the controller 71 are controlled by the controller 61, and the controller 61 is controlled by a control apparatus (for example, a cockpit domain controller) on a vehicle body side or a control apparatus on a sound box side.

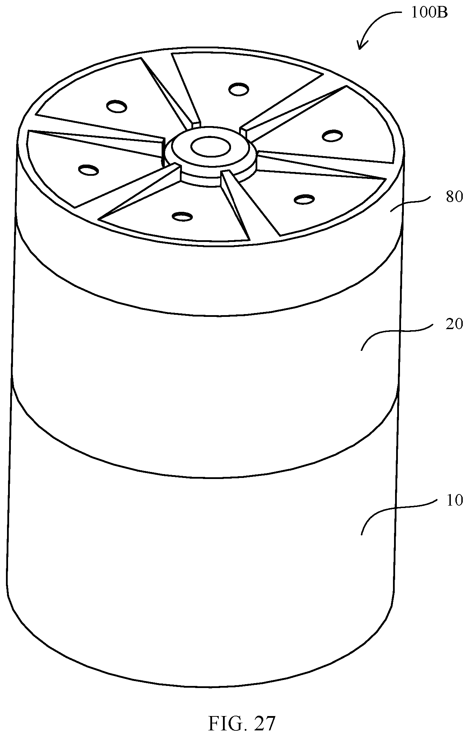

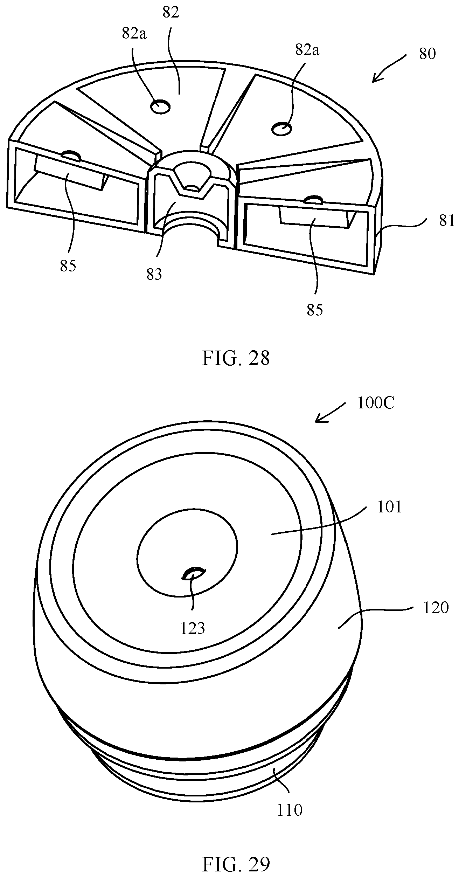

Refer to FIG. 27 and FIG. 28. The following describes an odor adjustment apparatus according to an embodiment of this application.

FIG. 27 is a three-dimensional schematic diagram of an odor adjustment apparatus according to an embodiment of this application. FIG. 28 is a schematic sectional view of an image playing unit according to an embodiment of this application.

A main difference between this embodiment and the foregoing embodiment lies in that, the odor adjustment apparatus 100B further includes an image playing unit 80, and other structures are the same as those in the foregoing embodiment. The same structures are represented by using same reference numerals, and detailed description thereof is omitted.

As shown in FIG. 27, the odor adjustment apparatus 100B includes a first housing 10, a second housing 20, and an image playing unit 80. The image playing unit 80 is mounted on the second housing 20, and is configured to play an image that uses a released aroma gas (aerosol) as a carrier. In this embodiment, the image playing unit 80 is a holographic projection unit, and can generate a three-dimensional image (holographic image).

As shown in FIG. 28, the image playing unit 80 includes a housing 81 and a plurality of projection mechanisms 85 mounted in the housing. The projection mechanism 85 is controlled by a controller 62 and is configured to generate a projected light beam. Light beams of the plurality of projection mechanisms 85 are combined to form a three-dimensional image.

Specifically, the housing 81 has a cover plate 82, the cover plate 82 is in an inverted cone shape, an outer surface of the cover plate 82 is a concave slope, and a plurality of window parts 82a are disposed on the cover plate 82. A plurality of projection mechanisms 85 are mounted on an inner surface of the cover plate 82, and the plurality of projection mechanisms 85 are in a one-to-one correspondence with locations of the plurality of window parts 82a, so that a light beam can be transmitted out of the housing 81 through the window parts 82a. The window part 82a may be a through hole that passes through the cover plate 82, or may be a transparent material part. In addition, the plurality of projection mechanisms 85 are configured as follows: Projection light beams of the projection mechanisms 85 are inclined towards a center side of the circular cover plate 82, and are converged together. In this way, reliable generation of a three-dimensional image can be ensured. In addition, 83 in FIG. 28 represents a through hole, and the through hole 83 communicates with the discharge hole 23, so that an aroma gas is diffused into an environmental space.

In the embodiment described above, the image playing unit 80 includes a housing 81 independent of the second housing 20. However, in another embodiment, the projection mechanism 85 may be disposed in the second housing 20, so that the housing 81 is omitted.

The following describes an odor adjustment apparatus according to an embodiment of this application with reference to FIG. 29 to FIG. 33.

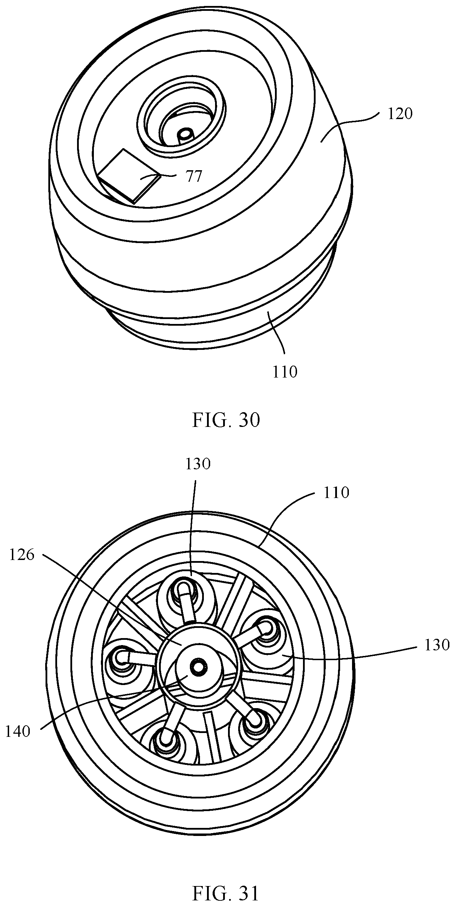

FIG. 29 is a three-dimensional schematic diagram of an odor adjustment apparatus according to an embodiment of this application. FIG. 30 is another three-dimensional schematic diagram of an odor adjustment apparatus according to an embodiment of this application. FIG. 31 is still another three-dimensional schematic diagram of an odor adjustment apparatus according to an embodiment of this application.

As shown in FIG. 29, the odor adjustment apparatus 100C has a housing 110, a housing 120, and a cover plate 101. Compared with the foregoing embodiment, the housing 120 is in an irregular shape, thereby improving aesthetics. The cover plate 101 is mounted on the housing 120, and a discharge hole 123 is disposed on the cover plate 101, and an aroma gas is diffused to an environmental space through the discharge hole 123. The cover plate 101 is removed from a state shown in FIG. 30. As shown in FIG. 30, a controller 77 (a circuit board) is disposed in a space between the cover plate 101 and the housing 120, and a light emitting lamp (not shown in the figure) is further disposed. In this embodiment, the cover plate 101 is made of a transparent or translucent material.

A state shown in FIG. 31 is a state in which the housing 120 is removed. As shown in FIG. 31, a plurality of spice containers 130 are mounted in the housing 110, and spice outlets of the plurality of spice containers 130 communicate with a mixing cavity 126, so that the spices can be released to the mixing cavity 126, and an aroma gas is formed in the mixing cavity 126. An air pump 140 is disposed in the mixing cavity 126. Driven by the air pump 140, a gas in the mixing cavity 126 is discharged into the environmental space. The air pump 140 corresponds to the blowing mechanism in this application.

The following describes a structure of the spice container.

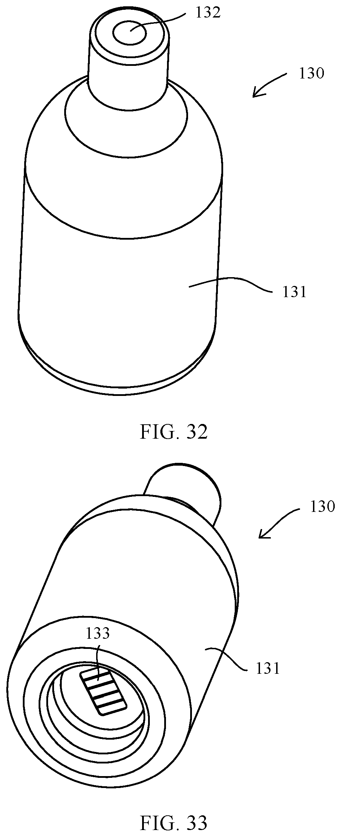

FIG. 32 is a three-dimensional schematic diagram of a spice container according to an embodiment of this application. FIG. 33 is another three-dimensional schematic diagram of a spice container according to an embodiment of this application.

In this embodiment, the spice container 130 is a spice bottle, and may be made of glass or plastic. The spice container 130 has a container body 131, and a film 132 is mounted on an opening (bottle opening) of the container body 131. The film 132 allows a spice to pass through, so that the spice can be diffused from the container body 131. The spice in the spice container 130 may be liquid, or may be solid or paste.

As shown in FIG. 33, on the spice container 130, specifically, a controller 133 (a circuit board and a chip) is fastened to a bottom of the bottle. The controller 133 records identification information, where the identification information indicates an odor type of the spice in the spice container 130, and the like. In addition, when the spice is a liquid, an ultrasonic atomization unit is disposed in the container body 131. The ultrasonic atomization unit is electrically connected to the controller 133 and is controlled by the controller 133, so that the liquid spice can be atomized and diffused from the container body 131. The plurality of ultrasonic atomization units and the plurality of controllers 133 correspond to the spice diffusing mechanism in this application, and the ultrasonic atomization unit and the controller 133 on the single spice container 130 correspond to the spice distributing unit in this application (the spice diffusing mechanism includes the plurality of spice distributing units).

## Claims

1. A control method for an odor adjustment system, comprising:
obtaining (S10) scene information;
determining (S20) a target odor type of a to-be-released gas based on the scene information; and
sending (S30) a control instruction, wherein the control instruction comprises a gas release instruction for releasing the gas of the target odor type,
**characterised in that**,
the scene information comprises schedule reminder information,
wherein the control method comprises:
obtaining the schedule reminder information, wherein the schedule reminder information comprises reminder content;
determining a first target odor type of a first to-be-released gas based on the reminder content; and
sending a first control instruction, wherein the first control instruction comprises a first gas release instruction for releasing the first to-be-released gas of the first target odor type,
wherein the schedule reminder information further comprises a reminder time; and
the control method further comprises:
obtaining current time information; and
sending the first control instruction when the reminder time matches the current time information.

2. The control method according to claim 1, wherein the scene information comprises one or more of driving fatigue scene information, user emotion information, off-odor removal scene information, driving mode information, music scene information, environment information, historical scene record information, or user-defined scene information.

3. The control method according to claim 1 or 2, further comprising: determining a target person based on the reminder content information; and
sending the first control instruction when the target person is detected.

4. The control method according to any one of claims 2 or 3, wherein when the scene information comprises the off-odor removal scene information, the control method comprises:
when the off-odor removal scene information is detected, sending a second control instruction, wherein the second control instruction comprises a second gas release instruction for releasing a deodorizing gas; and the off-odor removal scene information comprises information indicating one or more of the following: receiving a deodorization instruction of a user, receiving deodorization confirmation information of the user, detecting an off-odor, detecting a deodorization requirement, and detecting a first user action.

5. The control method according to any one of claims 1 to 4, further comprising: detecting verification information of a spice, and when the verification information meets a first condition, sending alarm information, wherein the first condition comprises one or more of the following: the verification information is inconsistent with preset verification information, and the verification information cannot be read.

6. A control apparatus (200A) for an odor adjustment system, comprising an obtaining module (250) and a processing module (240), wherein
the obtaining module (250) is configured to obtain scene information; and
the processing module (240) is configured to: determine a target odor type of a to-be-released gas based on the scene information, and send a control instruction, wherein the control instruction comprises a gas release instruction for releasing a gas of the target odor type,
**characterised in that**,
the scene information comprises schedule reminder information,
wherein the obtaining module (250) is configured to obtain the schedule reminder information, wherein the schedule reminder information comprises reminder content; and
the processing module (240) is configured to: determine a first target odor type of a first to-be-released gas based on the reminder content; and send a first control instruction, wherein the first control instruction comprises a first gas release instruction for releasing the first to-be-released gas of the first target odor type,
wherein the schedule reminder information further comprises a reminder time;
the obtaining module (250) is further configured to obtain current time information; and
the processing module (240) is configured to send the first control instruction when the reminder time matches the current time information.

7. The control apparatus according to claim 6, wherein the scene information comprises one or more of driving fatigue scene information, user emotion information, off-odor removal scene information, driving mode information, music scene information, environment information, historical scene record information, or user-defined scene information.

8. The control apparatus (200A) according to claim 6 or 7, wherein the processing module (240) is further configured to determine a target person based on the reminder content information; and
the processing module (240) is further configured to send the first control instruction when the target person is detected.

9. The control apparatus (200A) according to any one of claims 7 or 8, wherein when the scene information comprises the off-odor removal scene information, the processing module (240) is configured to:
when the off-odor removal scene information is detected, send a second control instruction, wherein the second control instruction comprises a second gas release instruction for releasing a deodorizing gas; and the off-odor removal scene information comprises information indicating one or more of the following: receiving a deodorization instruction of a user, receiving deodorization confirmation information of the user, detecting an off-odor, detecting a deodorization requirement, and detecting a first user action.

10. The control apparatus (200A) according to any one of claims 7 to 9, wherein the processing module (240) is further configured to: detect verification information of a the spice, and when the verification information meets a first condition, send alarm information, wherein the first condition comprises one or more of the following: the verification information is inconsistent with preset verification information, and the verification information cannot be read.

11. A computer-readable storage medium, wherein the computer-readable storage medium stores program instructions, and when the program instructions are executed by a computer, the computer is enabled to perform the control method according to any one of claims 1 to 5.

## Patentansprüche

1. Steuerungsverfahren für ein Geruchseinstellungssystem, umfassend:
Erlangen (S10) von Szeneninformationen;
Bestimmen (S20) eines Zielgeruchstypen eines freizugebenden Gases auf der Grundlage der Szeneninformationen; und
Senden (S30) einer Steuerungsanweisung, wobei die Steuerungsanweisung eine Gasfreigabeanweisung zum Freigeben des Gases des Zielgeruchstyps umfasst,
**dadurch gekennzeichnet, dass**,
die Szeneninformation eine Terminerinnerungsinformation umfasst,
wobei das Steuerungsverfahren Folgendes umfasst:
Erlangen der Terminerinnerungsinformationen, wobei die Terminerinnerungsinformationen Erinnerungsinhalt umfassen;
Bestimmen eines ersten Zielgeruchstyps eines ersten freizugebenden Gases auf der Grundlage des Erinnerungsinhalts; und
Senden einer ersten Steuerungsanweisung, wobei die erste Steuerungsanweisung eine erste Gasfreigabeanweisung zum Freigeben des ersten freizugebenden Gases des ersten Zielgeruchstyps umfasst,
wobei die Terminerinnerungsinformation ferner eine Erinnerungszeit umfasst; und
das Steuerungsverfahren ferner Folgendes umfasst:
Erlangen aktueller Zeitinformationen; und
Senden der ersten Steuerungsanweisung, wenn die Erinnerungszeit mit der aktuellen Zeitinformation übereinstimmt.

2. Steuerungsverfahren nach Anspruch 1, wobei die Szeneninformation eine oder mehrere der Szeneinformationen über Fahrermüdung, Emotionsinformation des Benutzers, Szeneinformationen zur Beseitigung von Fehlgerüchen, Fahrmodusinformationen, Musikszeneninformationen, Umgebungsinformationen, historische Szenenaufzeichnungsinformationen oder benutzerdefinierte Szeneninformationen umfasst.

3. Steuerungsverfahren nach Anspruch 1 oder 2, ferner umfassend:
Bestimmen einer Zielperson auf der Grundlage der Erinnerungsinhaltsinformationen; und
Senden der ersten Steuerungsanweisung, wenn die Zielperson detektiert wird.

4. Steuerungsverfahren nach einem der Ansprüche 2 oder 3, wobei, wenn die Szeneninformation die Beseitigung von Fehlgerüchen umfasst, das Steuerungsverfahren Folgendes umfasst:
wenn die Szeneninformation zur Beseitigung von Fehlgerüchen detektiert wird, Senden einer zweiten Steuerungsanweisung, wobei die zweite Steuerungsanweisung eine zweite Gasfreigabeanweisung zum Freigeben eines desodorierenden Gases umfasst; und die Szeneninformation zur Beseitigung von Fehlgerüchen Informationen umfasst, die eines oder mehrere der Folgenden anzeigen: Empfangen einer Desodorierungsanweisung eines Benutzers, Empfangen einer Desodorierungsbestätigung des Benutzers, Detektieren eines Fehlgeruchs, Detektieren einer Desodorierungsanforderung und Detektieren einer ersten Benutzeraktion.

5. Steuerungsverfahren nach einem der Ansprüche 1 bis 4, ferner umfassend: Detektieren von Verifizierungsinformationen eines Gewürzes und, wenn die Verifizierungsinformationen einen ersten Zustand erfüllen, Senden von Alarminformationen, wobei der erste Zustand eines oder mehrere der Folgenden umfasst: die Verifizierungsinformationen sind nicht mit den voreingestellten Verifizierungsinformationen konsistent und die Verifizierungsinformationen können nicht gelesen werden.

6. Steuervorrichtung (200A) für ein Geruchseinstellungssystem, umfassend ein Erlangungsmodul (250) und ein Verarbeitungsmodul (240), wobei
das Erlangungsmodul (250) zum Erlangen von Szeneninformationen konfiguriert ist; und
das Verarbeitungsmodul (240) konfiguriert ist, um: eine Bestimmung des Zielgeruchstyps eines freizugebenden Gases auf der Grundlage der Szeneninformation durchzuführen und eine Steuerungsanweisung zu senden, wobei die Steuerungsanweisung die Freigabe eines Gases des Zielgeruchstyps umfasst,
**dadurch gekennzeichnet, dass**,
die Szeneninformationen Terminerinnerungsinformationen umfassen,
wobei das Erlangungsmodul (250) zum Erlangen der Terminerinnerungsinformationen konfiguriert ist, wobei die Terminerinnerungsinformationen einen Erinnerungsinhalt umfassen; und
das Verarbeitungsmodul (240) konfiguriert ist, um: eine Bestimmung eines ersten Zielgeruchstyps eines ersten freizugebenden Gases auf der Grundlage des Erinnerungsinhalts durchzuführen; und eine erste Steuerungsanweisung zu senden, wobei die erste Steuerungsanweisung eine erste Steuerungsanweisung zur Freigabe des ersten freizugebenden Gases des ersten Zielgeruchstyps umfasst,
wobei die Terminerinnerungsinformationen ferner eine Erinnerungszeit umfassen;
das Erlangungsmodul (250) ferner zum Erlangen aktueller Zeitinformationen konfiguriert ist; und
das Verarbeitungsmodul (240) zum Senden der ersten Steuerungsanweisung konfiguriert ist, wenn die Erinnerungszeit mit der aktuellen Zeitinformation übereinstimmt.

7. Steuervorrichtung nach Anspruch 6, wobei die Szeneninformationen eine oder mehrere der Szeneinformationen über Fahrermüdung, Emotionsinformationen des Benutzers, Szeneinformationenen zur Beseitigung von Fehlgerüchen, Fahrmodusinformationen, Musikszeneninformationen, Umgebungsinformationen, historische Szenenaufzeichnungsinformationen oder benutzerdefinierte Szeneninformationen umfasst.

8. Steuervorrichtung (200A) nach Anspruch 6 oder 7, wobei das Verarbeitungsmodul (240) ferner zur Bestimmung einer Zielperson auf der Grundlage der Erinnerungsinhaltsinformationen konfiguriert ist; und
das Verarbeitungsmodul (240) ferner zum Senden der ersten Steuerungsanweisung konfiguriert ist, wenn die Zielperson detektiert wird.

9. Steuervorrichtung (200A) nach einem der Ansprüche 7 oder 8, wobei, wenn die Szeneninformationen die Beseitigung von Fehlgerüchen umfassen, das Verarbeitungsmodul (240) konfiguriert ist, um:
wenn die Szeneninformationen zur Beseitigung von Fehlgerüchen detektiert werden, Senden einer zweiten Steuerungsanweisung, wobei die zweite Steuerungsanweisung eine zweite Gasfreigabeanweisung zum Freigeben eines desodorierenden Gases umfasst; und die Szeneninformation zur Beseitigung von Fehlgerüchen Informationen umfasst, die eines oder mehrere der Folgenden anzeigen: Empfangen einer Desodorierungsanweisung eines Benutzers, Empfangen einer Desodorierungsbestätigung des Benutzers, Detektieren eines Fehlgeruchs, Detektieren einer Desodorierungsanforderung und Detektieren einer ersten Benutzeraktion.

10. Steuervorrichtung (200A) nach einem der Ansprüche 7 bis 9, wobei das Verarbeitungsmodul (240) ferner konfiguriert ist, um: Verifizierungsinformationen eines Gewürzes zu detektieren und, wenn die Verifizierungsinformationen einen ersten Zustand erfüllen, Alarminformationen zu senden, wobei der erste Zustand eines oder mehrere der Folgenden umfasst: die Verifizierungsinformationen sind nicht mit den voreingestellten Verifizierungsinformationen konsistent und die Verifizierungsinformationen können nicht gelesen werden.

11. Computerlesbares Speichermedium, wobei das computerlesbare Speichermedium Programmbefehle speichert und, wenn die Programmbefehle von einem Computer ausgeführt werden, der Computer fähig ist, das Verfahren nach einem der Ansprüche 1 bis 5 durchzuführen.

## Revendications

1. Procédé de commande pour un système d'ajustement d'arôme, comprenant :
l'obtention (S10) d'informations de scène ;
la détermination (S20) d'un type d'arôme cible d'un gaz à libérer sur la base des informations de scène ; et
l'envoi (S30) d'une instruction de commande, dans lequel l'instruction de commande comprend une instruction de libération de gaz pour libérer le gaz du type d'arôme cible,
**caractérisé en ce que**,
les informations de scène comprennent des informations de rappel de planification,
dans lequel le procédé de commande comprend :
l'obtention des informations de rappel de planification, dans lequel les informations de rappel de planification comprennent un contenu de rappel ;
la détermination d'un premier type d'arôme cible d'un premier gaz à libérer sur la base du contenu de rappel ; et
l'envoi d'une première instruction de commande, dans lequel la première instruction de commande comprend une première instruction de libération de gaz pour libérer le premier gaz à libérer du premier type d'arôme cible,
dans lequel les informations de rappel de planification comprennent également une heure de rappel ; et
le procédé de commande comprend également :
l'obtention d'informations temporelles actuelles ; et
l'envoi de la première instruction de commande lorsque l'heure de rappel correspond aux informations temporelles actuelles.

2. Procédé de commande selon la revendication 1, dans lequel les informations de scène comprennent l'une ou plusieurs des informations de scène de fatigue de conduite, des informations d'émotion de l'utilisateur, des informations de scène d'élimination de mauvaises odeurs, des informations de mode de conduite, des informations de scène musicale, des informations d'environnement, des informations d'enregistrement de scène historique ou des informations de scène définie par l'utilisateur.

3. Procédé de commande selon la revendication 1 ou 2, comprenant également : la détermination d'une personne cible sur la base des informations de contenu de rappel ; et
l'envoi de la première instruction de commande lorsque la personne ciblée est détectée.

4. Procédé de commande selon l'une quelconque des revendications 2 ou 3, dans lequel, lorsque les informations de scène comprennent les informations de scène d'élimination de mauvaises odeurs, le procédé de commande comprend :
lorsque les informations de scène d'élimination de mauvaises odeurs sont détectées, l'envoi d'une seconde instruction de commande, dans lequel la seconde instruction de commande comprend une seconde instruction de libération de gaz pour libérer un gaz désodorisant ; et les informations de scène d'élimination de mauvaises odeurs comprennent des informations indiquant l'un ou plusieurs des éléments suivants : la réception d'une instruction de désodorisation d'un utilisateur, la réception d'informations de confirmation de désodorisation de l'utilisateur, la détection d'une mauvaise odeur, la détection d'une exigence de désodorisation et la détection d'une première action de l'utilisateur.

5. Procédé de commande selon l'une quelconque des revendications 1 à 4, comprenant également : la détection d'informations de vérification d'une épice, et lorsque les informations de vérification remplissent une première condition, l'envoi d'informations d'alarme, dans lequel la première condition comprend l'un ou plusieurs des éléments suivants : les informations de vérification sont incompatibles avec des informations de vérification prédéfinies, et les informations de vérification ne peuvent pas être lues.

6. Appareil de commande (200A) pour un système d'ajustement d'arôme, comprenant un module d'obtention (250) et un module de traitement (240), dans lequel
le module d'obtention (250) est configuré pour obtenir des informations de scène ; et
le module de traitement (240) est configuré pour : déterminer un type d'arôme cible d'un gaz à libérer sur la base des informations de scène, et envoyer une instruction de commande, dans lequel l'instruction de commande comprend une instruction de libération de gaz pour libérer un gaz du type d'arôme cible, **caractérisé en ce que**,
les informations de scène comprennent des informations de rappel de planification,
dans lequel le module d'obtention (250) est configuré pour obtenir les informations de rappel de planification, dans lequel les informations de rappel de planification comprennent un contenu de rappel ; et
le module de traitement (240) est configuré pour : déterminer un premier type d'arôme cible d'un premier gaz à libérer sur la base du contenu de rappel ; et envoyer une première instruction de commande, dans lequel la première instruction de commande comprend une première instruction de libération de gaz pour libérer le premier gaz à libérer du premier type d'arôme cible, dans lequel les informations de rappel de planification comprennent également une heure de rappel ;
le module d'obtention (250) est en outre configuré pour obtenir des informations temporelles actuelles ; et
le module de traitement (240) est configuré pour envoyer la première instruction de commande lorsque l'heure de rappel correspond aux informations temporelles actuelles.

7. Appareil de commande selon la revendication 6, dans lequel les informations de scène comprennent l'une ou plusieurs des informations de scène de fatigue de conduite, des informations d'émotion de l'utilisateur, des informations de scène d'élimination de mauvaises odeurs, des informations de mode de conduite, des informations de scène musicale, des informations d'environnement, des informations d'enregistrement de scène historique ou des informations de scène définie par l'utilisateur.

8. Appareil de commande (200A) selon la revendication 6 ou 7, dans lequel le module de traitement (240) est en outre configuré pour déterminer une personne cible sur la base des informations de contenu de rappel ; et
le module de traitement (240) est en outre configuré pour envoyer la première instruction de commande lorsque la personne cible est détectée.

9. Appareil de commande (200A) selon l'une quelconque des revendications 7 ou 8, dans lequel, lorsque les informations de scène comprennent les informations de scène d'élimination de mauvaises odeurs, le module de traitement (240) est configuré pour :
lorsque les informations de scène d'élimination de mauvaises odeurs sont détectées, envoyer une seconde instruction de commande, dans lequel la seconde instruction de commande comprend une seconde instruction de libération de gaz pour libérer un gaz désodorisant ; et les informations de scène d'élimination de mauvaises odeurs comprennent des informations `indiquant l'un ou plusieurs des éléments suivants : la réception d'une instruction de désodorisation d'un utilisateur, la réception d'informations de confirmation de désodorisation de l'utilisateur, la détection d'une mauvaise odeur, la détection d'une exigence de désodorisation et la détection d'une première action de l'utilisateur.

10. Appareil de commande (200A) selon l'une quelconque des revendications 7 ou 9, dans lequel le module de traitement (240) est en outre configuré pour : détecter des informations de vérification d'une épice, et lorsque les informations de vérification remplissent une première condition, envoyer des informations d'alarme, dans lequel la première condition comprend l'un ou plusieurs des éléments suivants : les informations de vérification sont incompatibles avec des informations de vérification prédéfinies, et les informations de vérification ne peuvent pas être lues.

11. Support de stockage lisible par ordinateur, dans lequel le support de stockage lisible par ordinateur stocke des instructions de programme, et lorsque les instructions de programme sont exécutées par un ordinateur, l'ordinateur est en mesure de réaliser le procédé de commande selon l'une quelconque des revendications 1 à 5.
